# EUROPEAN PATENT APPLICATION

(11) **EP 0 257 391 A2**
(43) Date of publication of application: **02.03.1988**
(21) Application number: 87111335.3
(22) Date of filing: 05.08.1987
(51) Int. Cl.: C07D 413/06, A61K 31/42

(54) **5-Substituted-3-phenyl-3-(1H-imidazol-1-ylmethyl)-2-methylisoxazolidines**

(30) Priority: 27.08.1986 US 900851; 27.08.1986 US 900852; 27.08.1986 US 900853; 10.04.1987 US 36828; 10.04.1987 US 36829; 10.04.1987 US 36830; 10.04.1987 US 36832
(71) Applicant: FISONS CORPORATION, Rochester, NY 14603 (US)
(72) Inventor: Georgiev, Vassil Stefanov, Penfield New York 14526 (US); Mullen, George Byron, Avon New York 14414 (US)
(74) Representative: Wright, Robert Gordon McRae

(57) **Abstract**

5-Substituted-3-phenyl-3-(1H-imidazol-1-ylmethyl)-2-methylisoxazolidines are useful as antifungal agents.

## Description

### Background of the Invention

This invention pertains generally to substituted 2-methylisoxazolidines and more specifically to 5-substituted-3-phenyl-3-(1H-imidazol-1-ylmethyl)-2-methylisoxazolidines which are useful as antifungal agents.

### Brief Summary of the Invention

In accordance with this invention there are provided compounds of the formula: 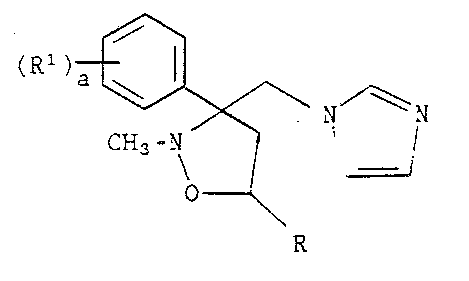 wherein;
a = 1 or 2,
R¹ is selected from hydrogen, lower alkyl, lower alkoxy, halogen, and combinations thereof, provided that the ortho position is hydrogen, and
R is selected from;
(a) 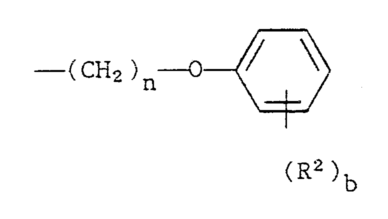 wherein;
   b = 1 or 2,
   n = 1 to 8, and
   R² is selected from hydrogen, lower alkyl, lower alkoxy, halogen, trifluoromethyl, nitro, acetamido, and combinations thereof,
(b) 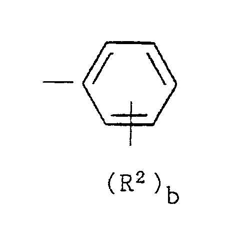 wherein;
   b = 1 or 2, and
   R² is selected from hydrogen, halogen, lower alkyl, lower alkoxy, and combinations thereof;
(c) 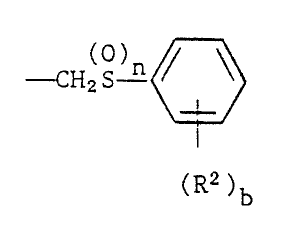 wherein;
   n = 0 to 2,
   b = 1 or 2, and
   R² is selected from hydrogen, halogen, lower alkyl, lower alkoxy, nitro, and cominations thereof;
(d) -(CH₂)ₙOR²
   wherein;
   n = 1 to 4, and
   R² is selected from hydrogen, lower alkyl, mono- or dihydroxy-substituted lower alkyl, allyl, cyclohexyl, lower alkyl-substituted cyclohexyl, methanesulfonyl and (halophenyl) methyl,
(e) 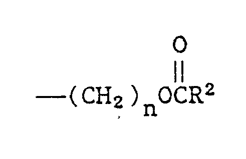 wherein;
   n = 1 to 4, and
   R² is selected from lower alkyl, phenyl, and mono- or disubstituted phenyl where the substituents on the phenyl ring are selected from halogen, lower alkyl, lower alkoxy, nitro, and combinations thereof,
(f) 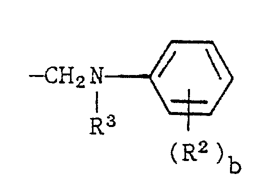 wherein;
   b = 1 or 2,
   R² is selected from hydrogen, lower alkyl, lower alkoxy, halogen, nitro, and combinations thereof, and R³ is selected from hydrogen, lower alkyl and benzyl, and
(g) phenylalkyl, substituted phenylalkyl, phenylalkenyl, substituted phenylalkenyl, C₁ to C₁₈ alkyl, (cycloalkyl)alkyl, and (alkoxycarbonyl)alkyl wherein the phenyl substituents are selected from one or more of lower alkyl, lower alkoxy, and halogen including combinations thereof,
and pharmaceutically acceptable acid addition salts thereof, in the form of their enantiomers, or mixtures of their enantiomers including diastereoisomeric pairs of such enantiomers.

### Detailed Description of the Invention

The compounds of this invention are useful as antifungal agents. They have been shown to have in vitro activity against yeast and systemic mycoses and dermatophytes as determined by broth and agar testing techniques [McGinnis, M.R., Laboratory Handbook Of Medical Mycology, Academic Press, N.Y., N.Y. (1980)]. The compounds have good to moderate inhibitory activity against a broad spectrum of organisms including trichophyton mentagrophytes, trichophyton tonsurans, trichophyton rubrum, trichophyton schoenleinii, epidermophyton floccosum, microsporum audouini, microsporum canis, candida albicans and candida stellatoidea (minimum inhibitory concentration, MIC, of < .2 to 70 ug/ml).

Because of the antifungal activity of the compounds of the invention they can be used, for example, in suitable liquid, semi-solid or solid carriers in the form of solutions, emulsions, suspensions, dispersions, ointments, aerosols, soaps, detergents, and powders in amounts effective to combat systemic and dermatophylic fungal infections in warm-blooded animals (1 to 20 percent active ingredient).

In the definition of the compounds of the invention, the alkyl moieties (CH₂)ₙ represent a branched or unbranched chain.

By halogen is meant chlorine, bromine, fluorine and iodine with chlorine and fluorine being preferred. By lower alkyl is meant C₁ to C₄ and by lower alkoxy is meant C₁ to C₆ which in either can be a branched or unbranched chain. By cycloalkyl(alkyl) is meant a C₁ to C₄ alkyl group substituted with a C₃ to C₈ cycloalkyl group and by (alkoxycarbonyl)alkyl is meant a C₁ to C₄ alkyl group substituted with a C₁-C₆ alkoxycarbonyl group. Compounds having ortho substitution of the 3-phenyl group were not prepared probably due to steric hindrance.

The 5-substituted-3-phenyl-3-(1H-imidazol-1-yl-methyl)-2-methylisoxazolidines are obtained as mixtures of cis- and trans-diastereomers due to the presence in the isoxazolidine ring of two asymmetric carbon atoms. The diastereomeric mixture is conveniently separated by flash-chromatography on silica gel using halogenated hydrocarbons (preferably dichloromethane and chloroform), alkanols (preferably methanol and ethanol), ethyl acetate and such as eluents. The eluents may be utilized alone or in combinations such as the ones comprised of 95-99% by volume halogenated hydrocarbon and 1-5% by volume alkanol. The stereochemistry of the two asymmetric carbon atoms in the isoxazolidine ring may be determined by conventional methods that include X-ray crystallography, nuclear magnetic resonance spectroscopy, circular dichroism or optical rotatory dispersion. Both the cis- and trans-diastereoisomers are resolvable into their optical enantiomers with (+)- and (-)-optical rotations by standard techniques such as fractional recrystallization of the diastereomeric salts with optically active organic acids such as (+)- and (-)-tartaric acid, (+)- and (-)-dibenzoyltartaric acid and the like.

As illustrated in the following diagrams, the compounds can be prepared starting with the reaction of appropriately substituted 2-(1H-imidazol-1-yl)acetophenones 1 with N-methylhydroxylamine hydrochloride in absolute ethanol at room or elevated (70-75°C) temperatures in the presence of base, for example, alkali metal carbonates, bicarbonates or acetates under a nitrogen atmosphere to give the corresponding nitrone derivatives 2. In one synthesis scheme, the latter derivative is treated with an appropriate allyl phenyl ether compound 3 to provide a diastereomeric mixture of the desired cis- and trans-substituted 5-(phenoxymethyl)-3-phenyl-3-(1H-imidazol-1-ylmethyl)-2-methylisoxazolidine derivative 4. Alternatively, reaction of the nitrone compound 2 with an appropriate 1-(ω-alkenyloxy)benzene derivative (5) leads to the corresponding diastereomeric mixture of cis- and trans-substituted 5-(ω-phenoxyalkyl)-3-phenyl-3-(1H-imidazol-1-ylmethyl)-2-methylisoxazolidine compound 6 (n>1).

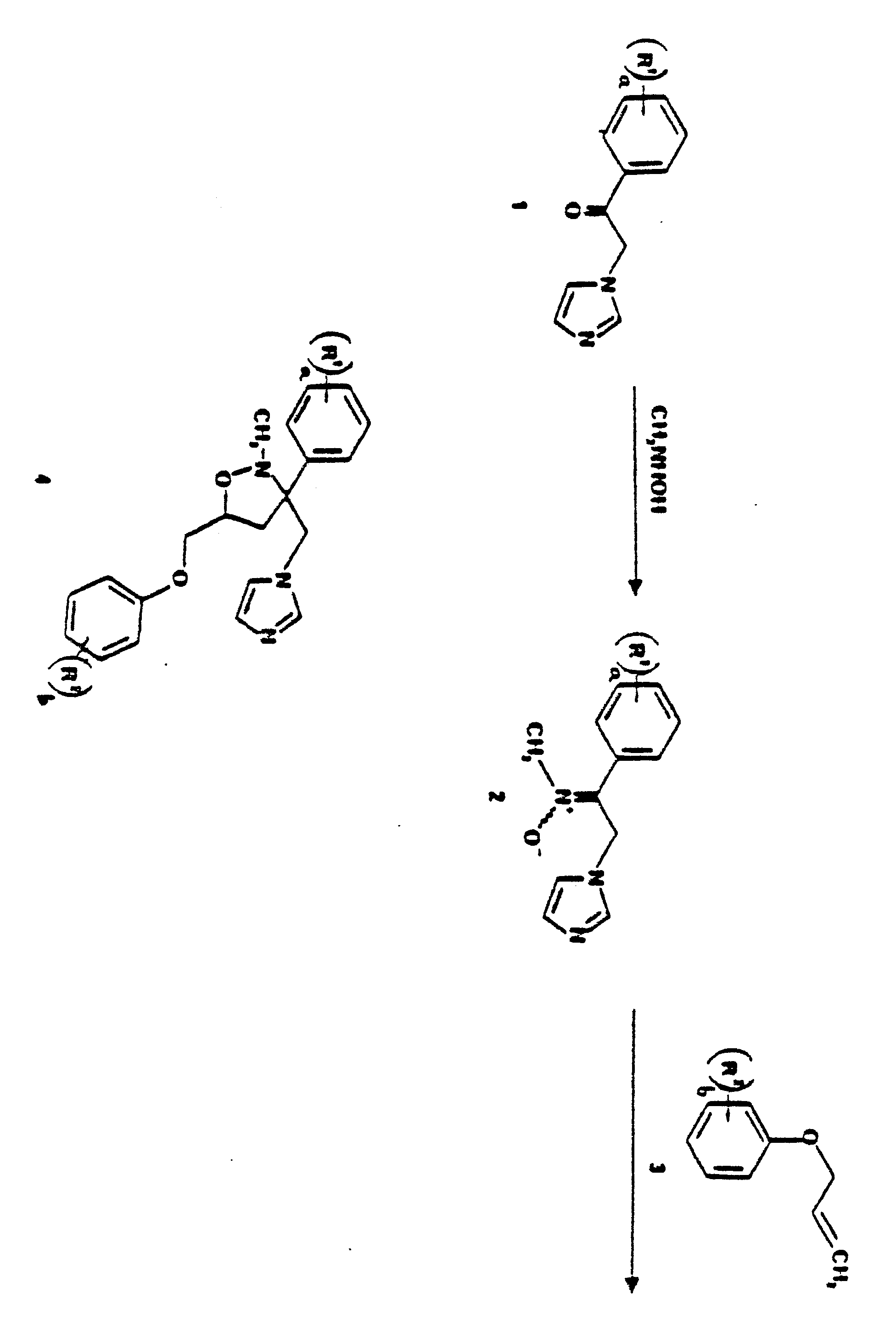

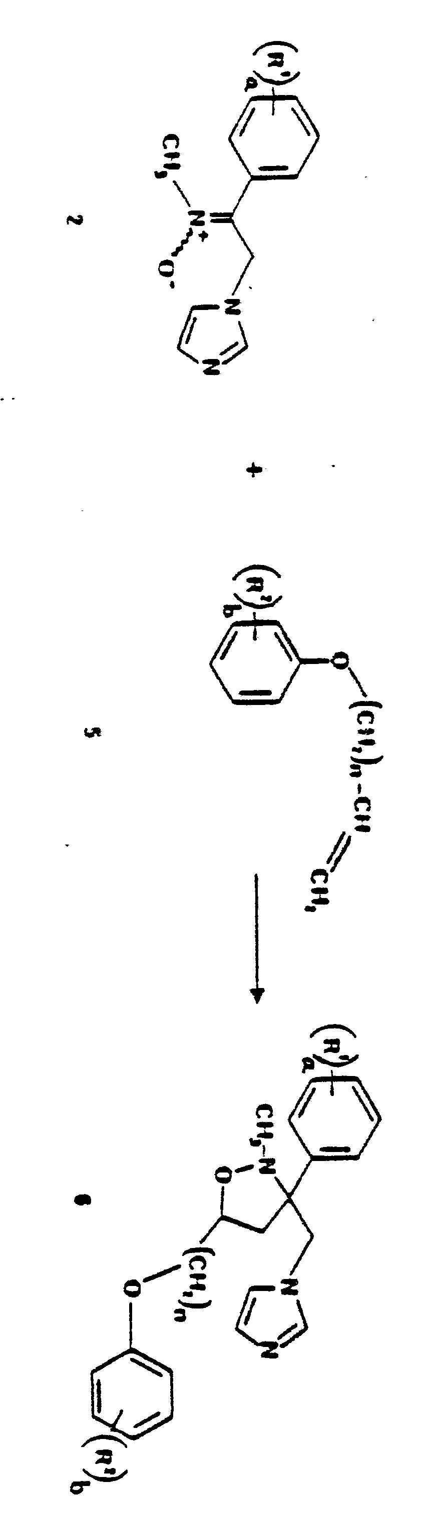

Similarly other compounds of the invention are prepared by reaction of the nitrone precursors 2 with:
(a) styrene derivatives 7 to give compounds 8; 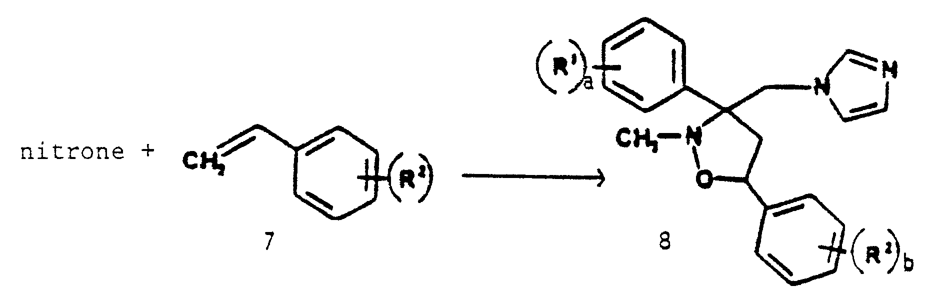
(b) allyl (substituted phenyl) sulfides 9 to give compounds 10, in which the sulfur could be optionally oxidized; 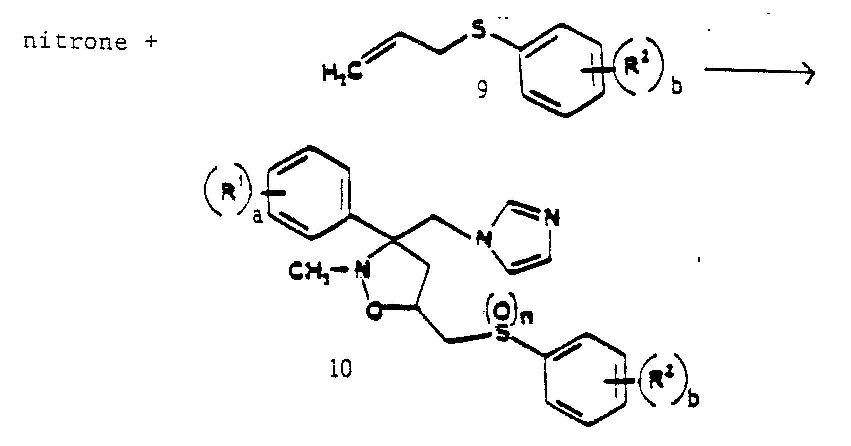
(c) 1-alkene alcohol or ether derivatives 11 or 14 having 3 to 6 carbon atoms to give compounds 12 and 15. When compounds 12 are treated with methane sulfonyl chloride the methanesulfonyloxy analogs 13 are prepared; 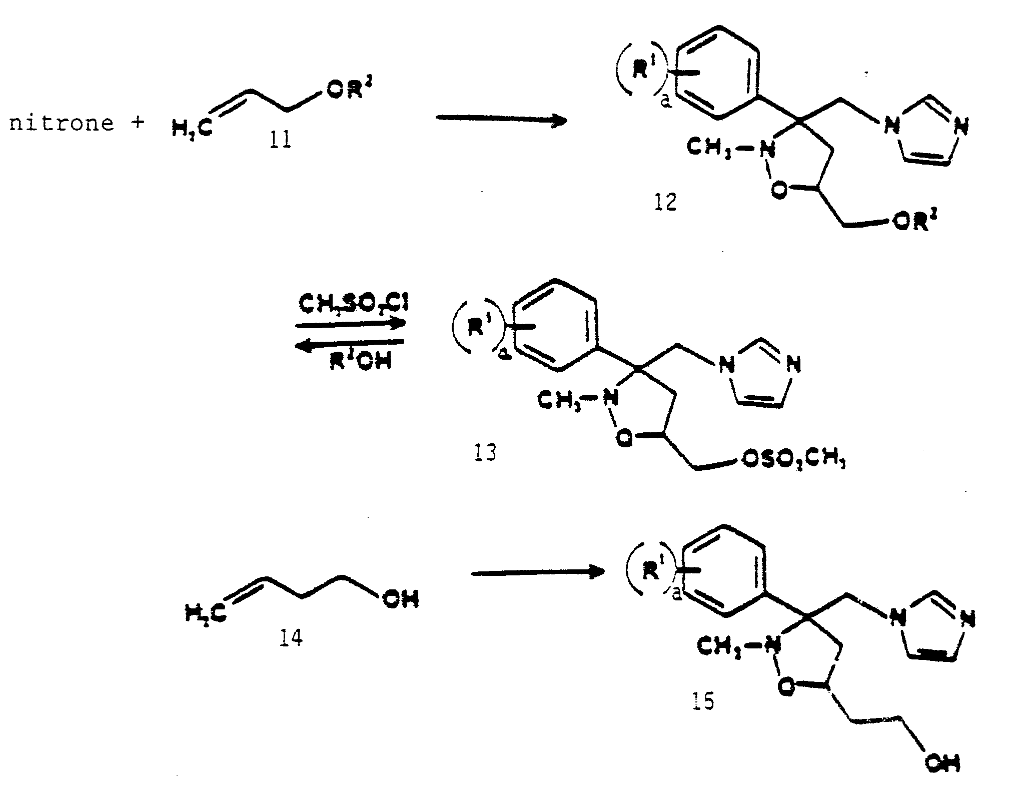
(d) 1-alkene alcohols 16 having three to six (3-6) carbons to give diasteriomeric mixtures of cis- and trans-alkanol compounds 17. Reaction of compounds 17 with acetic anhydride or an appropriate acyl chloride provides the esters 18; 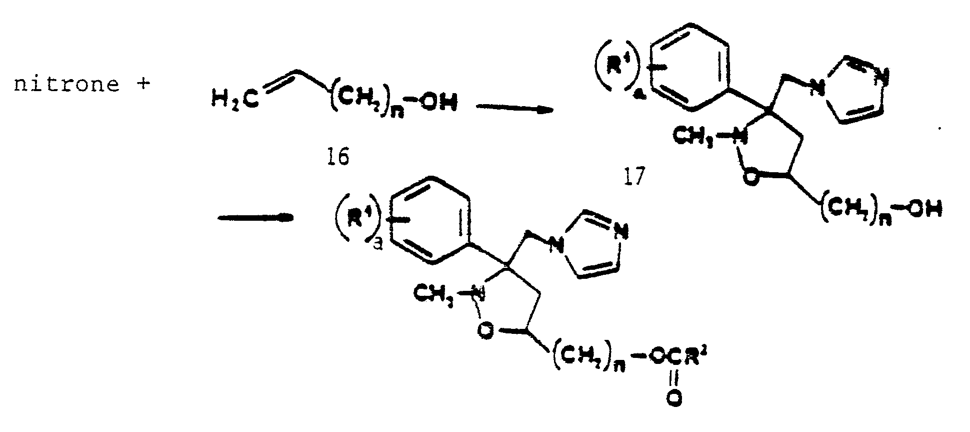
(e) 3-(phenylamino)prop-1-enes 19 to give compounds 20; 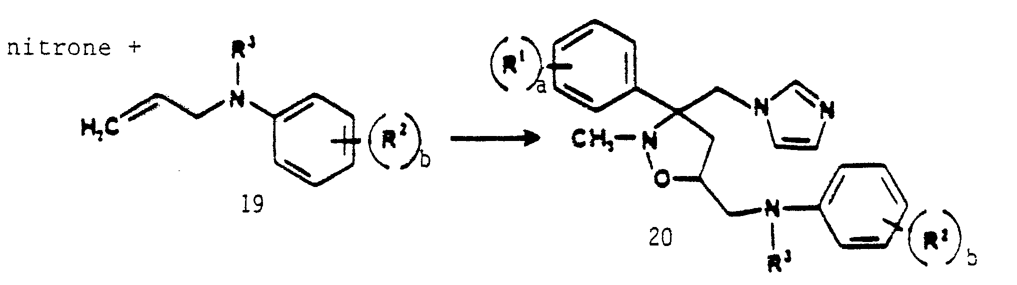
(f) 1-alkene derivatives 21 having 3 to 21 carbons in the carbon chain to give compounds 22.

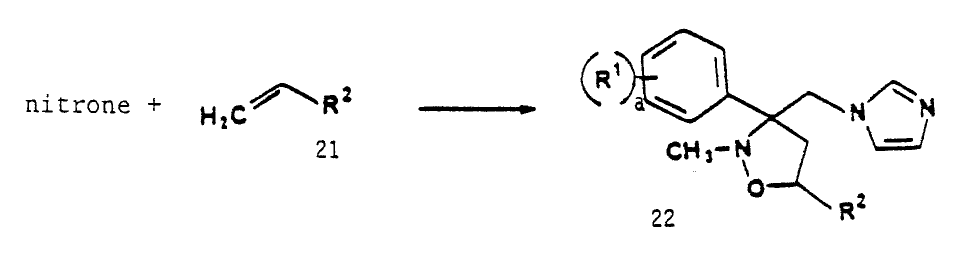

The compounds of the invention are basic and thus can form salts with pharmaceutically acceptable inorganic and organic acids such as, for example, acetic acid, maleic acid, malic acid, fumaric acid, succinic acid, succinamic acid, tartaric acid, citric acid, lactic acid, hydrochloric acid, hydrobromic acid, hydroiodic acid, nitric acid, sulfuric acid and phosphoric acid.

Salts of the compounds of the invention can be prepared as known in the art, for example, by dissolving the compound in a 10:1 by volume mixture of ethanol and aqueous acid such as HCl or HNO₃, evaporating the solvent, and then recrystallizing the crude salt, for example, from methanol-ether, 1:3 by volume in the case of HCl salts, and ethanol in the case of HNO₃ salts.

The preparation of the compounds of the invention is further illustrated by the following synthesis of intermediates and in the Examples.

### Preparation of Imidazolylacetophenones (1)

The imidazolylacetophenone compounds 1 are prepared according to known procedures, for example, (a) Godefroi et al., J. Medicinal chem. 12, 784 (1969) and (b) Nardi et al., J. Medicinal Chem. 24, 727 (1981).

The following imidazolylacetophenones 1 were synthesized:
(1) 2-(1H-imidazol-1-yl)acetophenone, mp 117-119°C,
(2) 2-(1H-imidazol-1-yl)-4ʹ-chloroacetophenone, mp 152-156°C,
(3) 2-(1H-imidazol-1-yl)-4ʹ-methylacetophenone, mp 133-137°C,
(4) 2-(1H-imidazol-1-yl)-4ʹ-methoxyacetophenone, mp 134-137°C,
(5) 2-(1H-imidazol-1-yl)-4ʹ-fluoroacetophenone, mp 150-155°C,
(6) 2-(1H-imidazol-1-yl)-3ʹ,4ʹ-dichloroacetophenone, mp 124-126°C,
(7) 2-(1H-imidazol-1-yl)-4ʹ-chloro-3ʹ-methylacetophenone, mp 116-118°C,
(8) 2-(1H-imidazol-1-yl)-3ʹ-methoxyacetophenone, mp 111-113°C,
(9) 2-(1H-imidazol-1-yl)-3ʹ-methylacetophenone.

### Preparation of Allyl Phenyl Ether Compounds (3)

The allyl phenyl ether compounds 3 are made by standard procedures [(a) White et al., J. Am. Chem. Soc. 80, 3271 (1958), (b) Tarbell and Wilson, J. Am. Chem. Soc. 64, 1066 (1942), (c) Mirek, Zesz, Nauk Univ. Jagiellon, Pr. Chem., No. 9, p. 77 (1964), and (d) McBee and Rapkin, J. Am. Chem. Soc. 73, 2375 (1951)] from appropriately substituted phenols and allyl bromide. The following allyl phenyl ether derivatives were prepared:
(1) allyl 4-chlorophenyl ether, bp 55-60°C (0.05 Torr),
(2) allyl 4-methylphenyl ether, bp 45-47°C (0.03 Torr),
(3) allyl 4-methoxyphenyl ether, bp 65-68°C (0.2 Torr),
(4) allyl 4-acetamidophenyl ether, mp 90-94°C,
(5) allyl 2-nitrophenyl ether, bp 92-99°C (0.15 Torr),
(6) allyl 2,4-dichlorophenyl ether, bp 62-65°C (0.05 Torr),
(7) allyl 2,6-dichlorophenyl ether, bp 45-50°C (0.05 Torr),
(8) allyl 4-fluorophenyl ether, bp 35-37°C (0.025 Torr), and
(9) allyl 3-(trifluoromethyl)phenyl ether, bp 35-37°C (0.2 Torr).

The 1-(3-butenyloxy)-4-chlorobenzene (5, R²=4-Cl, n = 2) was prepared by the procedure of Rius-Alonso and Wain, Ann. Apl. Biol. 88, 299 (1978), as were 4-chloro-1-(4-pentenyloxy)- benzene and 4-fluoro-1-(4-pentenyloxy) benzene.

### Preparation of Styrene Compounds

The styrene derivatives 7 are either commercially available, or may be prepared by standard procedures such as a Wittig reaction of an appropriately substituted benzaldehyde with methylenetriphenylphosphorane [Wittig and Schoellkopf, Chem. Ber. 87, 1318 (1954)]. All styrene derivatives 7 used in the synthesis of the 3,5-diphenyl-3-(1H-imidazol-1-ylmethyl)-2-methylisoxazolidines (8) in the Examples are commercially available.

### Preparation of Allyl (Substituted)phenyl Sulfides (9)

The allyl (substituted)phenyl sulfides 9 can be prepared by the method of Hurd and Greengard, J. Am. Chem. Soc., 52, 3356-3358 (1930).

The following allyl (substituted)phenyl sulfides 9 were synthesized.
(a) allyl phenyl sulfide, bp 65°C (0.75 mm),
(b) allyl 4-chlorophenyl sulfide, bp 90°C (0.15 mm),
(c) allyl 4-methylphenyl sulfide, bp 50-53°C (0.15 mm),
(d) allyl 4-nitrophenyl sulfide, bp 95°C (0.09 mm),
(e) allyl 4-fluorophenyl sulfide, bp 52-54°C (0.15 mm), and
(f) allyl 2-methoxyphenyl sulfide bp 77-80°C (0.50 mm).

### Preparation of Allyl Ethers:

Allyl alcohol (11: R² = H), 3-buten-1-ol (14) and 3-allyloxy-1,2-propanediol [11: R₂ = CH₂CH(OH)CH₂(OH)] are commercially available. The alkyl ethers (11) are prepared by standard literature procedures from appropriately substituted alkanol and allyl bromide, using sodium hydride as the base.
(a) allyl cyclohexyl ether (11: R² = c-C₆H₁₁ has a boiling point of 30-35°C (0.45 mm).
(b) allyl 4-tert-butylcyclohexyl ether [11: R² = c-C₆H₁₀C(CH₃)₃-4] has a boiling point of 55-60°C (0.40 mm), and
(c) allyl 4-chlorobenzyl ether (11 = R² = CH₂C₆H₄Cl-4) has a boiling point of 60-65°C (0.06 mm).

### Preparation of 3-(Phenylamino)prop-1-enes (19)

The synthesis of compounds 19 is accomplished by the procedures of Rodriguez and Canoira [J. Heterocyclic Chem., 22, 883-888 (1985)], and Laurent et al. [Synthesis (9), 685-700 (1983)].

### Example 1

### Preparation of 5-[(4-Chlorophenoxy)methyl]-3-(4-chlorophenyl)-3-(1H-imidazol-1-ylmethyl)-2-methylisoxazolidine (4, R¹=R²=4-Cl)

A suspension of 2-(1H-imidazol-1-yl)-4ʹ-chloroacetophenone (1, R¹=4-Cl) (19.90 g, 0.0902 mol), N-methylhydroxylamine hydrochloride (10.26 g, 0.123 mol) and sodium bicarbonate (10.37 g, 0.123 mol) in 200 ml ethanol is refluxed for 27 hours, under a nitrogen atmosphere. After cooling to room temperature, the suspension is filtered and the solvent is removed under reduced pressure. The residual orange-colored oil is dissolved in chloroform, filtered, and the solvent removed under reduced presure. The oily residue (compound 2, R¹=4-Cl) is taken up in 200 ml of toluene and 22.8 g (1.50 equivalent) of 4-chlorophenyl allyl ether (compound 3, R²=4-Cl) is added under a nitrogen atmosphere. The resulting solution is refluxed for 42 hours, cooled to room temperature and the solvent removed under reduced pressure. The residual dark-colored viscous oil which represents a cis- and trans-diastereomeric mixture of compound 4 (R¹=R²=4-Cl) is flash-chromatographed on neutral silica gel using chloroform and methanol (in a 98:2 ratio by volume) as eluent. 18.64 g (49.4%) of the cis-isomer A is obtained by crystallization from ether. An analytical sample is prepared by crystallization from ethyl acetate, mp 126-132°C. Anal. Calcd for C₂₁H₂₁Cl₂N₃O₂: C, 60.30; H, 5.06; N, 10.04; Cl, 16.95. Found: C, 60.36; H, 5.15; N. 9.97; Cl, 17.14. The trans-isomer B (6.28 g, 16.6%) is obtained by crystallization from isopropanol, mp 134-137°C. Anal. Calcd for C₂₁H₂₁Cl₂N₃O₂: C, 60.30; H, 5.06; N, 10.04: Cl, 16.95. Found: C, 60.36; H, 5.17; N, 9.97; Cl, 16.89.

The above preparation was repeated except that sodium acetate (20.18 g, 0.246 mol) was used to prepare the nitrone intermediate in place of sodium bicarbonate.

### Example 2

### 5-[(4-Chlorophenoxy)methyl]-3-(4-methoxyphenyl)-3-(1H-imidazol-1-ylmethyl)-2-methylisoxazolidine (4, R¹=4-OCH₃, R²=4-Cl)

The title compound 4 (R¹=4-OCH₃, R²=4-Cl) was made by a procedure similar to that described in Example 1 by reacting 2-(1H-imidazol-1-yl)-4ʹ-methoxyacetophenone (1, R¹=4-OCH₃) with N-methylhydroxylamine hydrochloride to form the corresponding nitrone derivative 2 (R¹=4-OCH₃), and treating the latter with 4-chlorophenyl allyl ether (3, R²=4-Cl). The resulting cis- and trans-diastereomeric mixture of compound 4 (R¹=4-OCH₃, R²=4-Cl) was flash-chromatographed on neutral silica gel using a 98:2 by volume mixture of chloroform and methanol as eluent. Isomer A has a melting point of 130-132°C (ethyl acetate). Anal. Calcd for C₂₂H₂₄ClN₃O₃: C, 63.84; H, 5.84: N, 10.15; Cl, 8.57. Found: C, 63.91: H, 5.94; N, 10.21; Cl, 8.59. Isomer B has a melting point of 36-38°C (ethyl acetate-hexane, in a 4:1 ratio by volume).

### Example 3

### 5-[(4-Chlorophenoxy)methyl]-3-(4-methylphenyl)-3-(1H-imidazol-1-ylmethyl)-2-methylisoxazolidine (4, R¹=4-CH₃, R²=4-Cl)

Compound 4 (R¹=4-CH₃, R²=4-Cl) was prepared by a procedure similar to that described in Example 1 by reacting 2-(1H-imidazol-1-yl)-4ʹ-methylacetophenone (1, R¹=4-CH₃) with N-methylhydroxylamine hydrochloride to form the corresponding nitrone derivative 2 (R¹=4-CH₃), and treating the latter with 4-chlorophenyl allyl ether (3, R²=4-Cl). The resulting cis- and trans-diastereomeric mixture of compound 4 (R¹=4-CH₃, R²=4-Cl) was purified by flash-chromatography on neutral silica gel using a 97:3 by volume mixture of chloroform and methanol as eluent. The pure isomer A (4, R¹=4-CH₃, R²=4-Cl) has a melting point of 143-145°C (ethyl acetate). Anal. Calcd for C₂₂H₂₄ClN₃O₂; C, 66.41; H, 6.08; N, 10.56; Cl, 8.91. Found: C, 66.38: H, 6.09; N. 10.54; Cl, 8.91.

### Example 4

### 5-[(4-Chlorophenoxy)methyl]-3-(4-fluorophenyl)-3-(1H-imidazol-1-ylmethyl)-2-methylisoxazolidine (4, R¹=4-F, R²=4-Cl)

Compound 4 (R¹=4-F, R²=4-Cl) was made by a procedure similar to that described in Example 1 by reacting 2-(1H-imidazol-1-yl)-4ʹ-fluoroacetophenone (1, R¹=4-F) with N-methylhydroxylamine hydrochloride to form the corresponding nitrone derivative 2 (R¹=4-F), and treating the latter with 4-chlorophenyl allyl ether (3, R²=4-Cl). The resulting cis- and trans-diastereomeric mixture of compound 4 (R¹=4-F, R²=4-Cl) was flash-chromatographed on neutral silica gel using a 98:2 by volume mixture of chloroform and methanol as eluent. Isomer A has a melting point of 125-127°C (ethyl acetate). Anal. Calcd for C₂₁H₂₁ClFN₃O₂: C, 62.76; H, 5.27; N, 10.46; Cl, 8.82. Found: C, 62.92; H, 5.33; N, 10.35; Cl, 8.78. Isomer B has a melting point of 61-65°C (isopropanol). Anal. Calcd for C₂₁H₂₁ClFN₃O₂: C, 62.76; H, 5.27; N, 10.46; Cl, 8.82. Found: C, 62.68; H, 5.50; N, 10.36; Cl, 8.86.

### Example 5

### 5-[(4-Chlorophenoxy)methyl]-3-(1H-imidazol-1-ylmethyl)-2-methylisoxazolidine (4, R¹=H, R²=4-Cl)

Compound 4 (R¹=H ,R²=4-Cl) was prepared by a procedure similar to that described in Example 1 by reacting 2-(1H-imidazol-1-yl)acetophenone (1, R¹=H) with N-methylhydroxylamine hydrochloride to form the corresponding nitrone derivative 2 (R¹=H), and treating the latter with 4-chlorophenyl allyl ether (3, R²=4-Cl). The resulting cis- and trans-diastereomeric mixture of compound 4 (R¹=H, R²=4-Cl) was flash-chromatographed on neutral silica gel using a 98:2 by volume mixture of chloroform and methanol as eluent. Isomer A has a melting point of 140-142°C (ethyl acetate). Anal. Calcd for C₂₁H₂₂ClN₃O₂: C, 65.71; H, 5.78; N, 10.95; Cl, 9.24. Found: C, 65.69; H, 5.84; N, 10.85; Cl, 9.57. Isomer B has a melting point of 141-143°C (ethyl acetate).

### Example 6

### 5-[(4-Chlorophenoxy)methyl]-3-(3,4-dichlorophenyl)-3-(1H-imidazol-1-ylmethyl)-2-methylisoxazolidine (4, R¹=3,4-Cl₂, R²=4-Cl)

Compound 4 (R¹=3,4-Cl₂,R²=4-Cl) was made by a procedure similar to that described in Example 1 by reacting 2-(1H- imidazol-1-yl)-3ʹ,4ʹ-dichloroacetophenone (1, R¹=3,4-Cl₂) with N-methylhydroxylamine hydrochloride to form the corresponding nitrone derivative 2 (R¹ = 3,4-Cl₂), and treating the latter with 4-chlorophenyl allyl ether (3, R²=4-Cl). The resulting cis- and trans-diastereomeric mixture of compound 4 (R¹ = 3,4-Cl₂,R²=4-Cl) was flash-chromatographed on neutral silica gel using a 98:2 by volume mixture of chloroform and methanol as eluent. Isomer A has a melting point of 149-151°C (ethyl acetate). Anal. Calcd for C₂₁H₂₀Cl₃N₃O₂: C, 55.71; H, 4.45; N, 9.28; Cl, 23.49. Found: C, 55.65; H, 4.50; N, 9.21; Cl, 23.61.

### Example 7

### 5-(Phenoxymethyl)-3-phenyl-3-(1H-imidazol-1-ylmethyl)-2-methylisoxazolidine (4, R¹=R²=H)

Compound 4 (R¹=R²=H) was obtained by a procedure similar to that described in Example 1 by reacting 2-(1H-imidazol-1-yl)acetophenone (1, R¹=H) with N-methylhydroxylamine hydrochloride to form the corresponding nitrone derivative 2 (R¹=H), and treating the latter with phenyl allyl ether (3, R²=H). The resulting cis- and trans-diastereomeric mixture of compound 4 (R¹=R²=H) was flash-chromatographed on neutral silica gel using a 98:2 by volume mixture of chloroform and methanol as eluent. Isomer A has a melting point of 107-109°C (ethyl acetate). Isomer B has a melting point of 165-168°C (isopropanol).

### Example 8

### 5-{[3-(Trifluoromethyl)phenoxy]methyl}-3-(4-chlorophenyl)-3-(1H-imidazol-1-ylmethyl)-2-methylisoxazolidine (4, R¹=4-Cl, R²=3-CF₃)

Compound 4 (R¹=4-Cl, R²=3-CF₃) was obtained by a procedure similar to that described in Example 1 by reacting 2-(1H- imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 3-(trifluoromethyl)phenyl allyl ether (3, R²=3-CF₃). The resulting cis-and trans-diastereomeric mixture of compound 4 (R¹=4-Cl, R²=3-CF₃) was flash-chromatographed on neutral silica gel using a 98:2 by volume mixture of dichloromethane and methanol as eluent. Isomer A has a melting point of 80-83°C (benzene). Anal. Calcd for C₂₂H₂₁N₃O₂ClF₃: C, 58.48; H, 4.68; N, 9.30; Cl, 7.85; F, 12.61. Found: C, 58.78; H, 4.60; N, 9.01; Cl, 7.84; F, 12.42.

### Example 9

### 5-[(4-Methoxyphenoxy)methyl]-3-(4-chlorophenyl)-3-(1H-imidazol-1-ylmethyl)-2-methylisoxazolidine (4, R¹=4-Cl, R²=4-OCH₃)

Compound 4 (R¹=4-Cl, R²=4-OCH₃) was prepared by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 4-methoxyphenyl allyl ether (3, R²=4-OCH₃). The resulting cis- and trans-diastereomeric mixture of compound 4 (R¹=4-Cl, R²=4-CH₃) was flash-chromatographed on neutral silica gel using a 98:2 by volume mixture of dichloromethane and methanol as eluent. Isomer A has a melting point of 140-145°C (ethyl acetate). Anal. Calcd for C₂₂H₂₄N₃O₃Cl: C, 63.84; H, 5.84; N, 10.15; Cl, 8.57. Found: C, 64.16; H, 5.95; N, 10.16; Cl, 8.60.

### Example 10

### 5-[(4-Methylphenoxy)methyl]-3-(4-chlorophenyl)-3-(1H-imidazol-1-ylmethyl)-2-methylisoxazolidine (4, R¹=4-Cl, R²=4-CH₃)

Compound 4 (R¹=4-Cl, R²=4-CH₃) was prepared by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 4-methylphenyl allyl ether (3, R²=4-CH₃). The resulting cis- and trans-diastereomeric mixture of compound 4 (R¹=4-Cl, R²=4-CH₃) was flash-chromatographed on neutral silica gel using a 98:2 by volume mixture of dichloromethane and methanol as eluent. Isomer A has a melting point of 115-123°C (ethyl acetate). Isomer B has a melting point of 111-134°C (ethyl acetate). Anal. Calcd for C₂₂H₂₄ClN₃O₂: C, 66.41; H, 6.08; N, 10.56; Cl, 8.91. Found C, 65.70; H, 6.15; N, 10.35; Cl, 9.10.

### Example 11

### 5-[2,6-Dichlorophenoxy)methyl]-3-(4-chlorophenyl)-3-(1H-imidazol-1-ylmethyl)-2-methylisoxazolidine (4, R¹=4-Cl, R²=2,6-Cl₂)

Compound 4 (R¹=4-Cl, R²=2,6-Cl₂) was made by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 2,6-dichlorophenyl allyl ether (3, R²=2,6-Cl₂). The resulting cis- and trans-diastereomeric mixture of compound 4 (R¹=4-Cl, R²=2,6-Cl₂) was flash-chromatographed on neutral silica gel using a 95:5 by volume mixture of dichloromethane and methanol as eluent. Isomer A has a melting point of 144-148°C (ethyl acetate). Anal. Calcd for C₂₁H₂₀N₃O₂Cl₃: C, 55.71; H, 4.45; N, 92.80; Cl, 23.49. Found: C, 55.72; H, 4.58; N, 9.22; Cl, 23.14.

### Example 12

### 5-[(2,4-Dichlorophenoxy)methyl]-3-(4-chlorophenyl)-3-(1H-imidazol-1-ylmethyl)-2-methylisoxazolidine (4, R¹=4-Cl, R²=2,4-Cl₂)

Compound 4 (R¹=4-Cl, R²=2,4-Cl₂) was obtained by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 2,4-dichlorophenyl allyl ether (3, R²=2,4-Cl₂). The resulting cis- and trans-diastereomeric mixture of compound 4 (R¹=4-Cl, R²=2,4-Cl₂) was flash-chromatographed on neutral silica gel using ethyl acetate as eluent. Isomer A has a melting point of 145-148°C (ethyl acetate). Anal. Calcd for C₂₁H₂₀N₃O₂Cl₃: C, 55.71; H, 4.45; N, 9.28; Cl, 23.49. Found C, 55.55; H, 4.51; N, 9.18; Cl, 23.32.

### Example 13

### 5-[(2-Nitrophenoxy)methyl]-3-(4-chlorophenyl)-3-(1H-imidazol-1-ylmethyl)-2-methylisoxazolidine (4, R¹=4-Cl, R²=2-NO₂)

Compound 4 (R¹=4-Cl, R²=2-NO₂) was made by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl) and treating the latter with 2-nitrophenyl allyl ether (3, R²=2-NO₂). The resulting cis- and trans-diastereomeric mixture of compound 4 (R¹=4-Cl, R²=2-NO₂) was flash-chromatographed on neutral silica gel using a 98:2 by volume mixture of dichloromethane and methanol as eluent. Isomer A has a melting point of 135-138°C (ethyl acetate). Anal. Calcd for C₂₁H₂₁N₄O₄Cl: C, 58.81; H, 4.94; N, 13.06; Cl, 8.27. Found: C, 58.88; H, 4.98; N, 13.12; Cl, 8.38.

### Example 14

### 5-[(4-Fluorophenoxy)methyl]-3-(4-chlorophenyl)-3-(1H-imidazol-1-ylmethyl)-2-methylisoxazolidine (4, R¹=4-Cl, R²=4-F)

Compound 4 (R¹=4-Cl, R²=4-F) was obtained by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 4-fluorophenyl allyl ether (3, R²=4-F). The resulting cis- and trans-diastereomeric mixture of compound 4 (R¹=4-Cl, R²=4-F) was flash-chromatographed on neutral silica gel using a 98:2 by volume mixture of dichloromethane and methanol as eluent. Isomer A has a melting point of 121-126°C (ethyl acetate). Calcd for C₂₁H₂₁N₃O₂ClF: C, 62.76; H, 5.27; N, 10.46; Cl, 8.82; F, 4.73. Found: C, 62.72; H, 5.26; N, 10.43; Cl, 8.66; F, 4.68.

### Example 15

### 5-[(4-Chlorophenoxy)methyl]-3-(4-chloro-3-methylphenyl)-3-(1H-imidazol-1-ylmethyl)-2-methylisoxazolidine (4,R¹=4-Cl, 3-CH₃, R²=4-Cl).

Compound 4 (R¹=4-Cl,3-CH₃, R²=4-Cl) was made by a procedure similar to that described in Example 1 by reacting 2-(1H-imidazol-1-yl)-4ʹ-chloro-3ʹ-methylacetophenone (1, R¹=4-Cl,3-CH₃) with N-methylhydroxylamine hydrochloride to form the corresponding nitrone derivative 2 (R¹=4-Cl,3-CH₃), and treating the latter with 4-chlorophenyl allyl ether (3, R²=4-Cl). The resulting cis- and trans-diastereomeric mixture of compound 4 (R¹=4-Cl,3-CH₃, R₂=4-Cl) was flash- chromatographed on neutral silica gel using a 98:2 by volume mixture of chloroform and methanol as eluent. Isomer A has a melting point of 142-146°C (ethyl acetate). Anal. Calcd for C₂₂H₂₃Cl₂N₃O₂: C, 61.12; H, 5.36; N, 9.72; Cl, 16.40. Found: C, 61.08; H, 5.45; N, 9.72; Cl, 16.34.

### Example 16

### 5-[(4-Chlorophenoxy)methyl]-3-(1H-imidazol-1-ylmethyl)-3-(3-methoxyphenyl)-2-methylisoxazolidine (4, R¹=3-CH₃O, R²=4-Cl)

Compound 4 (R¹=3-CH₃O, R²=4-Cl) was made by a procedure similar to that described in Example 1 by reacting 2-(1H-imidazol-1-yl)-3ʹ-methoxyacetophenone (1, R¹=-CH₃O) with N-methylhydroxylamine hydrochloride to form the corresponding nitrone derivative 2 (R¹=3-CH₃O) and treating the latter with 4-chlorophenyl allyl ether (3, R²=4-Cl). The resulting cis- and trans-diastereomeric mixture of compound 4 (R¹=3-CH₃O, R₂=4-Cl) was flash-chromatographed on neutral silica gel using a 98:2 by volume mixture of chloroform and methanol as eluent. Isomer A has a melting point of 92-95°C (ethyl ether). Anal. Calcd for C₂₂H₂₄ClN₃O₃: C, 63.84; H, 5.84; N, 10.15; Cl, 8.57. Found: C, 63.61; H, 5.90; N, 10.14; Cl, 8.55.

### Example 17

### 5-[(4-Chlorophenoxy)methyl]-3-(1H-imidazol-1-ylmethyl)-2-methyl-3-(3-methylphenyl)isoxazolidine (4, R¹=3-CH₃, R²=4-Cl)

Compound 4 (R¹=3-CH₃, R²=4-Cl) was made by a procedure similar to that described in Example 1 by reacting 2-(1H-imidazol-1- yl)-3ʹ-methylacetophenone (1, R¹=3-CH₃) with N-methylhydroxylamine hydrochloride to form the corresponding nitrone derivative 2 (R¹=3-CH₃), and treating the latter with 4-chlorophenyl allyl ether (3, R²=4-Cl). The resulting cis- and trans-diastereomeric mixture of compound 4 (R¹=3-CH₃, R²=4-Cl) was flash-chromatographed on neutral silica gel using a 99:1 by volume mixture of chloroform and methanol as eluent. Isomer A has a melting point of 122-124°C (ethyl acetate). Anal. Calcd for C₂₂H₂₄ClN₃O₂: C, 66.41; H, 6.08; N, 10.56; Cl, 8.91. Found: C, 66.45; H, 6.14; N, 10.63; Cl, 9.09.

### Example 18

### 5-[2-(4-Chlorophenoxy)ethyl]-3-(4-chlorophenyl)-3-(1H-imidazol-1-ylmethyl)-2-methylisoxazolidine (6, R¹=R²=4-Cl,n=2)

Compound 6 (R¹=4-Cl, R²=4-Cl, n=2) was prepared by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 1-(3-butenyloxy)-4-chlorobenzene (5, R²=4-Cl, n=2). The resulting cis- and trans-diastereomeric mixture of compound 6 (R¹=4-Cl, R²=4-Cl, n=2) was flash-chromatographed on neutral silica gel using a 98:2 by volume mixture of chloroform and methanol as eluent. Isomer A has a melting point of 103-105°C (ethyl acetate). Anal. Calcd for C₂₂H₂₃Cl₂N₃O₂: C, 61.12; H, 5.36; N, 9.72; Cl, 16.40. Found: C, 61.08; H, 5.44; N, 9.64; Cl, 16.50.

### Example 19

### 5-[3-(4-Chlorophenoxy)propyl]-3-(4-chlorophenyl)-3-(1H-imidazol-1-ylmethyl)-2-methylisoxazolidine (6, R¹=R²=4-Cl, n=3)

Compound 6 (R¹=R²=4-Cl, n=3) was prepared by a procedure similar to that described in Example 1 by reacting 2-(1H-imidazol-1-yl)-4ʹ-chloroacetophenone (1, R=4-Cl) with N-methylhydroxylamine hydrochloride to form the corresponding nitrone derivative 2 (R¹=4-Cl), and treating the latter with 1-(4-pentenyloxy)-4-chlorobenzene (5, R²=4-Cl, n=3). The resulting cis- and trans-diastereomeric mixture of compound 6 (R¹=R²=4-Cl, n=3) was flash-chromatographed on neutral silica gel using a 98:2 by volume mixture of chloroform and methanol as eluent. Isomer A has a melting point of 118-123°C (ethyl acetate). Anal. Calcd. for C₂₃H₂₅Cl₂N₃O₂: C, 61.89; H, 5.65; N, 9.41; Cl, 15.88. Found: C, 61.99; H, 5.71; N, 9.42; Cl, 15.47.

### Example 20

### 3-(4-Chlorophenyl)-5-[3-(4-fluorophenoxy)propyl]-3-(1H-imidazol-1-ylmethyl)-2-methylisoxazolidine (6, R¹=4-Cl, R²=4-F, n=3)

Compound 6 of (R¹=4-Cl, R²=4-F, n=3) was prepared by a procedure similar to that described in Example 1 by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 1-(4-pentenyloxy)-4-fluorobenzene (5, R²=4-F, n=3). The resulting cis- and trans-diastereomeric mixture of compound 6 (R¹=4-Cl, R²=4-F, n=3) was flash-chromatographed on neutral silica gel using a 98:2 by volume mixture of chloroform and methanol as eluent. Isomer A has a melting point of 116-120°C (ethyl acetate-hexane, 1:1 by volume).

### Example 21

### 5-[3-(4-Fluorophenoxy)propyl]-3-(4-fluorophenyl)-3-(1H-imidazol-1-ylmethyl)-2-methylisoxazolidine (6, R¹=R²=4-F,n=3)

Compound 6 (R¹=R²=4-F, n=3) was prepared by a procedure similar to that described in Example 1 by reacting 2-(1H-imidazol-1-yl)-4ʹ-fluoroacetophenone (1, R¹=4-F) with N-methylhydroxylamine hydrochloride to form the corresponding nitrone derivative 2 (R¹=4-F), and treating the latter with 1-(4-pentenyloxy)-4-fluorobenzene (5, R²=4-F, n=3). The resulting cis- and trans-diastereomeric mixture of compound 6 (R¹=R²=4-F, n=3) was flash-chromatographed on neutral silica gel using a 98:2 by volume mixture of chloroform and methanol as eluent. Isomer A has a melting point of 96-100°C (ethyl acetate-hexane, 1:1 by volume).

### Example 22

### 5-[(4-Fluorophenoxy)methyl]-3-(1H-imidazol-1-ylmethyl)-2-methyl-3-phenylisoxazolidine (4, R¹=H, R²=4-F)

Compound 4 (R¹=H, R²=4-F) was made by a procedure similar to that described in Example 1 by reacting 2-(1H-imidazol-1-yl)acetophenone (1, R¹=H) with N-methylhydroxylamine hydrochloride to form the corresponding nitrone derivative 2 (R¹=H), and treating the latter with 4-fluorophenyl allyl ether (3, R²=4-F). The resulting cis- and trans-diastereomeric mixture of compound 4 (R¹=H, R²=4-F) was flash-chromatographed on neutral silica gel using ethyl acetate as eluent. Isomer A has a melting point of 115-117°C (ethyl acetate). Anal. Calcd for C₂₁H₂₂FN₃O₂: C, 68.65; H, 6.04; N, 11.44; F, 5.17. Found: C, 68.54; H, 6.16; N, 11.41; F, 5.15. Isomer B has a melting point of 111-115°C (ethyl acetate). Anal. Calcd for C₂₁H₂₂FN₃O₂: C, 68.65; H, 6.04; N, 11.44; F, 5.17. Found: C, 69.03; H, 6.13; N, 11.38; F, 5.22.

### Example 23

### 5-[(4-Chloro-3-methylphenoxy)methyl]-3-(1H-imidazol-1-ylmethyl)-2-methyl-3-phenylisoxazolidine (4, R¹=H, R²=4-Cl,3-CH₃)

Compound 4 (R¹=H, R²=4-Cl, 3-CH₃) was prepared by a method similar to that described in Example 1 by reacting 2-(1H-imidazol-1-yl)acetophenone (1, R¹=H) with N-methylhydroxylamine hydrochloride to form the corresponding nitrone derivative 2 (R¹=H), and treating the latter with 4-chloro-3-methylphenyl allyl ether (3, R²=4-Cl,3-CH₃). The resulting cis- and trans-diastereomeric mixture of compound 4 (R¹=H, R²=4-Cl, 3-CH₃) was flash-chromatographed on neutral silica gel using a 98:2 by volume mixture of chloroform and methanol as eluent. Isomer A has a melting point of 110-120°C (ethyl acetate). Anal. Calcd for C₂₂H₂₄ClN₃O₂: C, 66.41; H, 6.08; N, 10.52; Cl, 8.91. Found: C, 66.38; H, 6.19; N, 10.46; Cl, 8.87.

### Example 24

### 3-(4-Fluorophenyl)-3-(1H-imidazol-1-ylmethyl)-2-methyl-5-phenoxymethyl)isoxazolidine (4, R¹=4-F, R²=H)

Compound 4 (R¹=4-F,R²=H) was made by a procedure similar to that described in Example 1 by reacting 4ʹ-fluoro-2-(1H-imidazol-1-yl)acetophenone (1, R¹=4-F) with N-methylhydroxylamine hydrochloride to form the corresponding nitrone derivative 2 (R¹=4-F), and treating the latter with phenyl allyl ether (3, R²=H). The resulting cis- and trans-diastereomeric mixture of compound 4 (R¹=4-F, R²=H) was flash-chromatographed on neutral silica gel using a 98:2 by volume mixture of chloroform and methanol. Isomer A has a melting point of 164-166°C (isopropanol). Anal. Calcd for C₂₁H₂₂FN₃O₂: C, 68.65; H, 6.04; N, 11.44; F, 5.17. Found: C, 68.62; H, 5.96; N, 11.37; F, 5.12.

### Example 25

### 5-[(4-Fluorophenoxy)methyl]-3-(4-fluorophenyl)-3-(1H-imidazol-1-ylmethyl)-2-methylisoxazolidine (4, R¹=R²=4-F)

Compound 4 (R¹=R²=4-F) was made by a procedure similar to that described in Example 1 by reacting 4ʹ-fluoro-2-(1H-imidazol-1-yl)acetophenone (1, R¹=4-F) with N-methylhydroxylamine hydrochloride to form the corresponding nitrone derivative 2 (R¹=4-F), and treating the latter with 4-fluorophenyl allyl ether (3, R²=4-F). The resulting cis- and trans-diastereomeric mixture of compound 4 (R¹=R²=4-F) was flash-chromatographed on neutral silica gel using ethyl acetate as the eluant. Isomer A has a melting point 141-143°C (ethyl acetate). Anal. Calcd for C₂₁H₂₁F₂N₃O₂: C, 65.44; H, 5.49; N, 10.90; F, 9.86. Found: C, 65.84; H, 5.38; N, 10.88; F, 9.55.

### Example 26

### 3-(1H-Imidazol-1-ylmethyl)-5-[(4-methoxyphenoxy)methyl]-3-(4-methoxyphenyl)-2-methylisoxazolidine (4, R¹=R²=4-OCH₃)

Compound 4 (R¹=R²=4-OCH₃) was made by a procedure similar to that described in Example 1 by reacting 2-(1H-imidazol-1-yl)-4ʹ-methoxyacetophenone (1, R¹=4-OCH₃) with N-methylhydroxylamine hydrochloride to form the corresponding nitrone derivative 2 (R¹=4-OCH₃), and treating the latter with 4-methoxyphenyl allyl ether (3, R²=4-OCH₃). The resulting cis- and trans-diastereomeric mixture of compound 4 (R¹=R²=4-OCH₃) was flash-chromatographed on neutral silica gel using a 98:2 by volume mixture of methylene chloride and methanol as eluent. Isomer A has a melting point of 96-101°C (ethyl acetate). Anal. Calcd for C₂₃H₂₇FN₃O₄: C, 67.46; H, 6.65; N, 10.26. Found C, 67.43; H, 6.64; N, 10.20.

### Example 27

### 5-[(4-Acetamidophenoxy)methyl]-3-(4-chlorophenyl)-3-(1H-imi dazol-1-ylmethyl)-2-methylisoxazolidine (4, R¹=4-Cl, R²=NHAc)

Compound 4 (R¹=4-Cl, R²=NHAc) was prepared by a method similar to that described in Example 1 by reacting 4ʹ-chloro-2-(1H-imidazol-1-yl)acetophenone (1, R¹=4-Cl) with N-methylhydroxylamine hydrochloride to form the corresponding nitrone derivative 2 (R¹=4-Cl), and treating the latter with 4-acetamidophenyl allyl ether (3, R²=4-NHAc). The resulting cis- and trans-diastereomeric mixture of compound 4 (R¹=4-Cl, R²=4-NHAc) was flash-chromatographed on neutral silica gel using a 98:2 by volume mixture of chloroform and methanol as eluent. Isomer A has a melting point of 181-186°C (ethyl acetate). Anal. Calcd for C₂₃H₂₅ClN₄O₃: C, 62.65; H, 5.72; N, 12.71; Cl, 8.04. Found: C, 62.52; H, 5.76; N, 12.58; Cl, 8.09.

### Example 28

### Salts of 5-[(4-Chlorophenoxy)methyl]-3-(4-chlorophenyl)-3-(1H-imidazol-1-ylmethyl)-2-methylisoxazolidine (4, R¹=R²=4-Cl)

Salts of the title compound 4 were prepared by dissolving the compound in a 10:1 by volume mixture of ethanol and the appropriate concentrated acid, evaporating the solvent and then recrystallizing the crude salt from methanol-ether (1:3 by volume in the case of the HCl salt), and ethanol in the case of the NHO₃ salt. Isomer A · 2HCl has a melting point of 170-183°C. Anal. Calcd for C₂₁H₂₃Cl₄N₃O₂: C, 51.35; H, 4.72; N, 8.55; Cl, 28.87. Found: C, 51.45; H, 4.84; N, 8.50; Cl, 27.72. Isomer A ·HNO₃ has a melting point of 165-167°C. Anal. Calcd for C₂₁H₂₂Cl₂N₄O₅: C, 52.40; H, 4.61; N, 11.67; Cl, 14.73. Found: C, 52.51; H, 4.70; N, 11.67; Cl, 14.84.

### Example 29

### 3,5-Bis(4-methoxyphenyl)-3-(1H-imidazol-1-ylmethyl)-2-methylisoxazolidine (8, R¹=R²=4-OCH₃)

Under a nitrogen atmosphere, a suspension of 21.75 g (0.101 mol) of 2-(1H-imidazol-1-yl)-4ʹ-methoxyacetophenone (1, R¹=4-OCH₃), 11.89 g (0.142 mol) of N-methylhydroxylamine hydrochloride and 12.03 g (0.143 mol) of sodium bicarbonate in 250 ml of absolute ethanol was heated to reflux and stirred for 45 hours. After cooling to room temperature, the suspension was filtered and the solvent removed in vacuo. The residual orange oil, containing nitrone 2 (R¹=4-OCH₃) was taken up in chloroform, filtered and the solvent removed in vacuo. The oil was then dissolved in 250 ml of toluene, under a nitrogen atmosphere, and 25.0 g (0.186 mol) of 4-methoxystyrene (7, R²=4-OCH₃) was added. The resulting solution was refluxed for 23 hours, cooled to room temperature, and the solvent removed in vacuo. The crude cis- and trans-diastereomeric mixture of compound 8 (R¹=R² =4-OCH₃) was purified by flash-chromatography on neutral silica gel using ethyl acetate as eluent. Isomer A has a melting point of 117-120°C (ethyl acetate). Anal. Calcd. for C₂₂H₂₅N₃O₃: C, 69.64; H, 6.64; N, 11.07. Found: C, 69.57; H, 6.85; N, 10.88.

### Example 30

### 3,5-Diphenyl-3-(1H-imidazol-1-ylmethyl)-2-methylisoxazolidine (8, R¹=R²=H)

Compound 8 (R¹=R²=H) was prepared by a procedure similar to that described in Example 29, by reacting 2-(1H-imidazol-1-yl)acetophenone (1, R¹=H) with N-methylhydroxylamine hydrochloride to form the corresponding nitrone derivative 2 R¹=H), and treating the latter with styrene (7, R²=H). The resulting cis- and trans-diastereomeric mixture of compound 8 (R¹=R²=H) was purified by flash-chromatography on neutral silica gel using ethyl acetate as eluent. Isomer A has a melting point of 97-99°C (ethyl ether). Anal. Calcd. for C₂₀H₂₁N₃O: C, 75.21; H, 6.63; N, 13.16. Found: C, 75.14; H, 6.71; N, 13.14. Isomer B has a melting point of 200-202°C (methanol). Anal. Calcd. for C₂₀H₂₁N₃O: C, 75.21; H, 6.63; N, 13.16. Found: C 75.07; H, 6.73; N, 13.12.

### Example 31

### 3-(4-Chlorophenyl)-3-(1H-imidazol-1-ylmethyl)-2-methyl-5-phenylisoxazolidine (8, R¹=4-Cl, R²=H)

Compound 8 (R¹=4-Cl, R²=H) was prepared by a procedure similar to that described in Example 29, by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with styrene (7, R²=H). The resulting cis- and trans-diastereomeric mixture of compound 8 (R¹=4-Cl, R²=H) was purified by flash-chromatography on neutral silica gel using a 98:2 by volume mixture of chloroform and methanol as eluent. Isomer A has a melting point of 159-163°C (ethyl acetate). Anal. Calcd. for C₂₀H₂₀ClN₃O: C, 67.89; H, 5.70; N, 11.88; Cl, 10.02. Found: C, 67.78; H, 5.80; N, 11.83; Cl, 9.94. Isomer B has a melting point of 124-127°C (ethyl acetate-hexane, 1:1 by volume).

### Example 32

### 3,5-Bis(4-chlorophenyl)-3-(1H-imidazol-1-ylmethyl)-2-methylisoxazolidine (8, R¹=R²=4-Cl)

Compound 8 (R¹=R²=4-Cl) was prepared by a procedure similar to that described in Example 29, by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and reacting the latter with 4-chlorostyrene (7, R²=4-Cl). The resulting cis- and trans-diastereomeric mixture of compound 8 (R¹=R²=4-Cl) was purified by extraction of the toluene solution with 2 N hydrochloric acid, neutralization of the aqueous acid layer with solid potassium carbonate, and back-extraction with chloroform. Isomer A has melting point of 137-139°C (ethyl acetate). Anal. Calcd. for C₂₀H₁₉Cl₂N₃O: C, 61.87; H, 4.93; N, 10.82. Found: C, 61.73; H, 4.99; N, 10.81. The preparation of the title compound 4 (R¹=R²=4-Cl) was repeated except that the nitrone 2 was prepared using sodium acetate rather than sodium bicarbonate.

### Example 33

### 5-(2-Chlorophenyl)-3-(4-chlorophenyl)-3-(1H-imidazol-1-yl-methyl)-2-methylisoxazolidine (8, R¹=4-Cl, R²=2-Cl)

Compound 8 (R¹=4-Cl, R²=2-Cl) was prepared by a procedure similar to that described in Example 29, by reacting 2-(1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 2-chlorostyrene (7, R¹=2-Cl). The resulting cis- and trans-diastereomeric mixture of compound 8 (R¹=4-Cl, R²=2-Cl) was purified by flash-chromatography on neutral silica gel using a 95:5 by volume mixture of chloroform and methanol as eluent. Isomer A has a melting point of 129-132°C (ethyl acetate). Anal. Calcd. for C₂₀H₁₉Cl₂N₃O: C, 61.87; H, 4.93; N, 10.82. Found: C, 61.77; H, 5.00; N, 10.77.

### Example 34

### 3-(4-Chlorophenyl)-5-(2,6-dichlorophenyl)-3-(1H-imidazol-1-ylmethyl)-2-methylisoxazolidine (8, R¹=4-Cl, R²=2,6-Cl₂)

Compound 8 (R¹=4-Cl, R²=2,6-Cl₂) was prepared by a procedure similar to that described in Example 29, by reacting 2-(1H-imidazol-1-yl)-4ʹ-chloroacetophenone (1, R¹= 4-Cl) with N-methylhydroxylamine hydrochloride to form the corresponding nitrone derivative 2 (R¹=4-Cl), and treating the latter with 2,6-dichlorostyrene (7, R²=2,6-Cl₂). The resulting cis- and trans-diastereomeric mixture of compound 8 (R¹=4-Cl, R²=2,6-Cl₂) was purified by flash-chromatography on neutral silica gel using ethyl acetate as eluent. Isomer A has a melting point of 159-161°C (ethyl acetate). Anal. Calcd. for C₂₀H₁₈Cl₃N₃O: C, 56.82; H, 4.29; N, 9.94. Found: C, 56.90; H, 4.50; N, 9.73. Isomer B, has a melting point of 64-66°C (isopropanol) as its 1:1 complex with isopropanol. Anal. Calcd. for C₂₀H₁₈Cl₃N₃O.C₃H₈O: C, 57.21; H, 5.43; N, 8.70; Cl, 22.03. Found: C, 57.20; H, 5.40; N, 8.74; Cl, 22.12.

### Example 35

### 3-(4-Chlorophenyl)-3-(1H-imidazol-1-ylmethyl)-2-methyl-5-(4-methylphenyl)isoxazolidine (8, R¹=4-Cl, R²=4-CH₃)

Compound 8 (R¹=4-Cl, R²=4-CH₃) was prepared by a procedure similar to that described in Example 29, by reacting (1H-imidazol-1-yl)-4ʹ-chloroacetophenone (1, R¹=4-Cl) with N-methylhydroxylamine hydrochloride to form the corresponding nitrone derivative 2 (R¹=4-Cl), and treating the latter with 4-methylstyrene (7, R²=4-CH₃). The resulting cis- and trans-diastereomeric mixture of compound 8, (R¹=4-Cl, R²=4-CH₃) was purified by flash-chromatography on neutral silica gel using a 98:2 by volume mixture of chloroform and methanol as eluent. Isomer A has a melting point of 135.5-137°C (ethyl acetate). Anal. Calcd. for C₂₁H₂₂ClN₃O: C, 68.56; H, 6.03; N, 11.42; Cl, 9.64. Found: C, 68.40; H, 6.10; N, 11.26; Cl, 9.67. Isomer B has a melting point of 129°C (ethyl acetate). Anal. Calcd. for C₂₁H₂₂ClN₃O: C, 68.56; H, 6.03; N, 11.42; Cl, 9.64. Found: C, 68.67; H, 6.14; N, 11.44; Cl. 10.06.

### Example 36

### 3-(4-Fluorophenyl)-3-(1H-imidazol-1-ylmethyl)-2-methyl-5-phenylisoxazolidine (8, R¹=4-F, R²=H)

Compound 8 (R¹=4-F, R²=H) was prepared by a procedure similar to that described in Example 29, by reacting 2-(1H-imidazol-1-yl)-4ʹ-fluoroacetophenone (1, R¹=4-F) with N-methylhydroxylamine hydrochloride to form the corresponding nitrone derivative 2 (R¹=4-F), and treating the latter with styrene (7, R²=H). The resulting cis- and trans-diastereomeric mixture of compound 8 (R¹=4-F, R²= H) was purified by flash-chromatography on neutral silica gel using a 98:2 by volume mixture of chloroform and methanol as eluent. Isomer A has a melting point of 130-132°C (ethyl acetate). Anal. Calcd. for C₂₀H₂₀FN₃O: C, 71.20; H, 5.97; N, 12.45; F, 5.63. Found: C, 71.33; H, 6.52; N, 12.46; F, 5.64.

### Example 37

### 5-(3,4-Dimethoxyphenyl)-3-(1H-imidazol-1-ylmethyl)-2-methyl-3-phenylisoxazolidine [8, R¹=H, R²=3,4-(OCH₃)₂]

Compound 8 [R¹=H, R²=3,4-(OCH₃)₂] was prepared by a procedure similar to that described in Example 29, by reacting 2-(1H-imidazol-1-yl)acetophenone (1, R¹=H) with N-methylhydroxylamine hydrochloride to form the corresponding nitrone derivative 2 (R¹=H), and treating the latter with 3,4-dimethoxystyrene [7, R²= 3,4-(OCH₃)₂]. The resulting cis- and trans-diastereomeric mixture of compound 8 [R¹=H, R²=3,4-(OCH₃)₂] was purified by flash-chromatography on neutral silica gel using a 98:2 by volume mixture of chloroform and methanol as eluent. Isomer A has a melting point of 136-144°C (isopropanol) as its monohydrochloride salt which was prepared by dissolving the compound in an ethanol-concentrated HCl mixture (10:1 by volume), evaporating the solvent and then recrystallizing the acid salt from methanol-ether, 1:3 by volume.

### Example 38

### 3-(3-Methoxyphenyl)-3-(1H-imidazol-1-ylmethyl)-2-methyl-5-{[(4-chlorophenyl)thio]methl}isoxazolidine (10, R¹=3-CH₃O, R²=4-Cl, n=0)

A solution of 29.4 g (0.12 mol) of 2-(1H-imidazol-1-yl)-1-(3-methoxyphenyl)-N-methylethanimine N-oxide (2, R¹=3-OCH₃) [prepared by reacting 2-(1H-imidazol-1-yl)-3ʹ-methoxyacetophenone (24.03 g, 0.120 mol) with N-methylhydroxylamine hydrochloride (12.03 g, 0.144 mol) and sodium bicarbonate (12.10 g, 0.144 mol) in 300 ml of ethanol)] and 29.5 g (0.16 mol) of allyl 4-chlorophenyl sulfide (9, R²=4-Cl) in 300 ml of toluene was refluxed for 24 hours under a nitrogen atmosphere. Then, the reaction mixture was cooled to room temperature and the solvent evaporated under reduced pressure. The crude cis- and trans-diastereomeric mixture of the title compound was flash-chromatographed on neutral silica gel using ethyl acetate as the eluent. Isomer A (5.69 g) has a melting point of 113-114°C (ethyl acetate). Anal. Calcd for C₂₂H₂₄ClN₃O₂S: C, 61.46; H, 5.63; N, 9.77; Cl, 8.25; S, 7.46. Found: C, 61.35; H, 5.65; N, 9.71; Cl, 8.59; S, 7.57.

### Example 39

### 3-(4-Chlorophenyl)-3-(1H-imidazol-1-ylmethyl)-2-methyl-5-[(phenylthio)methyl]isoxazolidine (10, R¹=4-Cl,R²=H, n=0)

The title compound was prepared by a procedure similar to that described in Example 38 by reacting 1-(4-chlorophenyl)-2-(1H-imidazol-1-yl)-N-methylethanimine N-oxide (2, R¹=4-Cl) with allyl phenyl sulfide (9, R²=H). The resulting cis- and trans-diastereomeric mixture of the title compound was flash-chromatographed on neutral silica gel using ethyl acetate as eluent. Isomer A has a melting point of 81-88°C (ethyl acetate-petroleum ether, 1:1 by volume). Anal. Calcd. for C₂₁H₂₂ClN₃OS: C, 63.07; H, 5.54; N, 10.51; Cl, 8.86. Found: C, 62.98; H, 5.62; N, 10.44; Cl, 9.15. Isomer B has a melting point of 81-85°C (ether).

### Example 40

### 3-(3-Methoxyphenyl)-3-(1H-imidazol-1-ylmethyl)-2-methyl-5-{[(4-methylphenyl)thio]methyl}isoxazolidine (10, R¹=3-OCH₃, R²=4-CH₃, n=0

The title compound was prepared by a procedure similar to that described in Example 38 by reacting 2-(1H-imidazol-1-yl)-1-(3-methoxyphenyl)-N-methylethanimine N-oxide (2, R¹=3-OCH₃) with allyl 4-methyphenyl sulfide (9: R²=4-CH₃). The resulting cis- and trans-diastereomeric mixture of compound 10 (R¹ = 3-OCH₃, R² = 4-CH₃, n = 0) was flash-chromatographed on neutral silica gel using ethyl acetate as eluent. Isomer A has a melting point of 107-108°C (ethyl acetate). Anal. Calcd for C₂₃H₂₇N₃O₂S: C, 67.45; H, 6.65; N, 10.26; S, 7.83. Found: C, 67.37; H, 6.59; N, 10.22; S, 8.02.

### Example 41

### 3-(4-Chlorophenyl)-3-(1H-imidazol-1-ylmethyl)-2-methyl-5-{[(4-methylphenyl)thio]methyl}isoxazolidine (10: R¹=4-Cl, R²=4-CH₃, n=0)

Compound 10 (R¹=4-Cl, R²=4-CH₃, n=0) was prepared by a procedure similar to that described in Example 38 by reacting 1-(4-chlorophenyl)-2-(1H-imidazol-1-yl)-N-methylethanimine N-oxide (2, R¹=4-Cl) with allyl 4-methylphenyl sulfide (9, R²=4-CH₃). The resulting cis- and trans-diastereomeric mixture of the title compound was flash-chromatographed on neutral silica gel using ethyl acetate as eluent. Isomer A has a melting point of 98-100°C (ethyl acetate). Anal. Calcd for C₂₂H₂₄ClN₃OS: C, 64.00; H, 5.84; N, 10.15; Cl, 8.56; S, 7.75. Found: C, 63.98; H, 5.98; N, 10.11; Cl, 8.68; S, 7.84. The preparation was repeated except that nitrone 2 was prepared using sodium acetate in place of sodium bicarbonate.

### Example 42

### 3-(4-Chlorophenyl)-3-(1H-imidazol-1-ylmethyl)-2-methyl-5-{[(4-fluorophenyl)thio]methyl}isoxazolidine (10, R¹=4-Cl, R²=4-F, n=0)

Compound 10 (R¹=4-Cl, R²=4-F, n=0) was prepared by a procedure similar to that described in Example 38 by reacting 1-(4-chlorophenyl)-2-(1H-imidazol-1-yl)-N-methylethanimine N-oxide (2, R¹=4-Cl) with allyl 4-fluorophenyl sulfide (9, R²=4-F). The resulting cis- and trans-diastereomeric mixture of the title derivative was flash-chromatographed on neutral silica gel using ethyl acetate as eluent. Isomer A has a melting point of 106-107°C (ethyl acetate-hexane, 1:1 by volume). Anal. Calcd for C₂₁H₂₁ClFN₃OS: C, 60.35; H, 5.06; N, 10.05; Cl, 8.48; F, 4.55; S, 7.67. Found: C, 60.08; H, 4.84; N, 9.99; Cl, 8.57; F, 4.61; S, 8.02.

### Example 43

### 3-(4-Chlorophenyl)-3-(1H-imidazol-1-ylmethyl)-2-methyl-5-{[(4-nitrophenyl)thio]methyl}isoxazolidine (10, R¹=4-Cl, R²=4-NO₂, n=0)

Compound 10 (R¹=4-Cl, R²=4-NO₂,n=0) was prepared by a procedure similar to that described in Example 38 by reacting 1-(4-chlorophenyl)-2-(1H-imidazol-1-yl)-N-methylethanimine N-oxide (2, R¹=4-Cl) with allyl 4-nitrophenyl sulfide (9, R²=4-NO₂). The resutling cis- and trans-diastereomeric mixture of the title compound was flash-chromatographed on neutral silica gel using a 98:2 by volume mixture of chloroform and methanol as eluent. Isomer A has a melting point of 179-180°C (ethanol). Anal. Calcd for C₂₁H₂₁ClN₄O₃S: C, 56.69; H, 4.76; N, 12.59; Cl, 7.97; S, 7.21. Found: C, 56.47; H, 4.90; N, 12.39; Cl, 8.21; S, 7.46.

### Example 44

### 3-(4-Chlorophenyl)-3-(1H-imidazol-1-ylmethyl)-2-methyl-5-{[(4-chlorophenyl)thio]methyl}isoxazolidine (10, R¹=R²=4-Cl, n=0)

Compound 10 (R¹=R²=4-Cl, n=0) was prepared according to a procedure similar to that described in Example 38 by reacting 1-(4-chlorophenyl)-2-(1H-imidazol-1-yl)-N-methylethanimine N-oxide (2, R¹=4-Cl) with allyl 4-chlorophenyl sulfide (9, R²=4-Cl). The resulting cis- and trans-diastereomeric mixture of compound 10 (R¹=R²=4-Cl, n=0) was flash-chromatographed on neutral silica gel using a 99:1 by volume mixture of chloroform and methanol as eluent. Isomer A has a melting point 118-119°C (ethyl acetate). Anal. Calcd for C₂₁H₂₁Cl₂N₃OS: C, 58.07; H, 4.87; N, 9.67; Cl, 16.32; S, 7.38. Found: C, 58.12; H, 4.95; N, 9.67; Cl, 16.29; S, 7.64.

### Example 45

### 3-Phenyl-3-(1H-imidazol-1-ylmethyl)-2-methyl-5-{[(4-chlorophenyl)thio]methyl}isoxazolidine (10, R¹=H, R²=4-Cl, n=0)

Compound 10 (R¹=H, R²=4-Cl, n=0) was prepared by a procedure similar to that described in Example 38 by reacting 1-phenyl-2-(1H-imidazol-1-yl)-N-methylethanimine N-oxide (2, R¹=H) with allyl 4-chlorophenyl sulfide (9, R²=4-Cl). The resulting cis- and trans-diastereomeric mixture of the title derivative was flash-chromatographed on neutral silica gel using chloroform-methanol (98:2 by volume) as eluent. Isomer A has a melting point of 132.5-133°C (ethyl acetate). Anal. Calcd for C₂₁H₂₂ClN₃OS: C, 63.07, H, 5.54; N, 10.51; Cl, 8.86; S, 8.02. Found: C, 62.93; H, 5.55; N, 10.45; Cl, 9.32; S, 8.50.

### Example 46

### 3-Phenyl-3-(1H-imidazol-1-ylmethyl)-2-methyl-5-{[(4-methylphenyl)thio]methyl}isoxazolidine (10, R¹=H, R²=4-CH₃, n=0)

Compound 10 (R¹=H, R²=4-CH₃, n=0) was prepared according to a procedure similar to that described in Example 38 by reacting 1-phenyl-2-(1H-imidazol-1-yl)-N-methylethanimine N-oxide (2, R¹=H) with allyl 4-methylphenyl sulfide (9, R²=4-CH₃). The resulting cis- and trans-diastereomeric mixture of the title compound was flash-chromatographed on neutral silica gel using chloroform-methanol (98:2 by volume) as eluent. Isomer A has a melting point of 116°C (ethyl acetate). Anal. Calcd for C₂₂H₂₅N₃OS: C, 69.63; H, 6.64; N, 11.07; S, 8.45. Found: C, 69.53; H, 6.69; N, 11.01; S, 8.54.

### Example 47

### 3-(4-Fluorophenyl)-3-(1H-imidazol-2-ylmethyl)-2-methyl-5-{[(4-chlorophenyl)thio]methyl}isoxazolidine (10, R¹=4-F, R²=4-Cl, n=0)

Compound 10 (R¹=4-F, R²=4-Cl, n=0) was prepared by a procedure similar to that described in Example 38 by reacting 1-(4-fluorophenyl)-2-(1H-imidazol-1-yl)-N-methylethanimine N-oxide (2, R¹=4-F) with allyl 4-chlorophenyl sulfide (9, R²=4-Cl). The resulting cis- and trans-diastereomeric mixture of compound 10 (R¹=4-F, R²=4-Cl, n=0) was flash-chromatographed on neutral silica gel using ethyl acetate as eluent. Isomer A has a melting point of 120.5°C (ethyl acetate). Anal. Calcd for C₂₁H₂₁ClFN₃OS: C, 60.35; H, 5.06; N, 10.05; Cl, 8.48; F, 4.55; S, 7.67. Found: C, 60.30; H, 5.12; N, 10.04; Cl, 8.48; F, 4.43; S, 7.88. The above preparation was repeated except that nitrone 2 was prepared using sodium acetate in place of sodium bicarbonate.

### Example 48

### 3-(4-Fluorophenyl)-3-(1H-imidazol-1-ylmethyl)-2-methyl-5-{[(4-methylphenyl)thio]methyl}isoxazolidine (10, R¹=4-F, R²=4-CH₃, n=0)

Compound 10 (R¹=4-F, R²=4-CH₃, n=0) was prepared by a procedure similar to that described in Example 38 by reacting 1-(4-fluorophenyl)-2-(1H-imidazol-1-yl)-N-methylethanimine N-oxide (2, R¹=4-F) with allyl 4-methylphenyl sulfide (9, R²=4-CH₃). The resulting cis- and trans-diastereomeric mixture of compound 10 (R¹=4-F, R²=4-CH₃, n=0) was flash-chromatographed on neutral silica gel using a 99:1 by volume mixture of chloroform and methanol as eluent. Isomer A has a melting point of 109.5-110°C (ethyl acetate). Anal. Calcd for C₂₂H₂₄FN₃OS: C, 66.47; H, 6.09; N, 10.57; F, 4.78; S, 8.07. Found: C, 66.39; H, 6.10; N, 10.69; F, 5.02; S, 8.24.

### Example 49

### 3-(3,4-Dichlorophenyl)-3-(1H-imidazol-1-ylmethyl)-2-methyl-5-{[(2-methoxyphenyl)thio]methyl}isoxazolidine (10, R¹=3,4-Cl₂, R²=2-OCH₃, n=0)

Compound 10 (R¹=3,4-Cl₂, R²=2-OCH₃, n=0) was prepared according to a procedure similar to that described in Example 38 by reacting 1-(3,4-dichlorophenyl)-2-(1H-imidazol-1-yl)-N-methylethanimine N-oxide (2, R¹=3,4-Cl₂) with allyl 2-methoxyphenyl sulfide (9, R²=2-OCH₃). The resulting cis- and trans-diastereomeric mixture of compound 10 (R¹=3,4-Cl₂, R²=2-OCH₃, n=0) was flash-chromatographed on neutral silica gel using ethyl acetate as eluent. Isomer A has a melting point of 55-65°C (decomp.) (ethyl acetate). Anal. Calcd for C₂₂N₂₃Cl₂N₃O₂S: C, 56.90; H, 4.99; N, 9.05; Cl, 15.27; S, 6.90. Found: C, 56.53; H, 5.20; N, 8.89; Cl, 15.10; S, 7.08.

### Example 50

### 3-(3-Methoxyphenyl)-3-(1H-imidazol-1-ylmethyl)-2-methyl-5-{[(4-chlorophenyl)sulfoxyl]methyl}isoxazolidine (10, R¹=OCH₃, R²=4-Cl, n=1)

Under a nitrogen atmosphere, a solution of 2.0 g (4.65 mmol) of 3-(3-methoxyphenyl)-3-(1H-imidazol-1-ylmethyl)-2-methyl -5-{[(4-chlorophenyl)thio]methyl}isoxazolidine (10, R¹=3-OCH₃, R²=4-Cl, n=0, isomer A) in 100 ml of methylene chloride was cooled to -78°C using a dry ice-acetone bath. To this solution was added dropwise over a period of 30 minutes, a solution of 1.24 g (5.75 mmol) of 85% m-chloroperbenzoic acid in 70 ml of methylene chloride. The resulting solution was warmed gradually to room temperature then washed repeatedly with saturated aqueous sodium bicarbonate solution (3 x 100 ml) and dried over anhydrous magnesium sulfate. The solvent was evaporated under reduced pressure and the crude product was flash-chromatographed on neutral silica gel using a 98:2 by volume mixture of chloroform and methanol as eluent. A pure compound 10 (R¹=3-OCH₃, R²=4-Cl, n=1) (0.43 g, 21% yield) was obtained as white crystals. Mp 120-121°C (ethyl acetate). Anal. Calcd for C₂₂H₂₄ClN₃O₃S: C, 59.25; H, 5.42; N, 9.42; S, 7.19. Found: C, 58.96; H, 5.52; N, 9.32; S, 7.41.

### Example 51

### 3-(4-Chlorophenyl)-3-(1H-imidazol-1-ylmethyl)-2-methyl-5-{[(4-chlorophenyl)sulfoxyl]methyl}isoxazolidine (10, R¹=R²=4-Cl, n=1)

Compound 10 (R¹=R²=4-Cl, n=1) was prepared by a procedure similar to that described in Example 50 by reacting 3-(4-chlorophenyl)-3-(1H-imidazol-1-ylmethyl)-2-methyl-5-{[(4- chlorophenyl)thio]methyl}isoxazolidine (10, R¹=R²=4-Cl, n=0, isomer A) with 85% m-chloroperbenzoic acid. The resulting crude compound 10 (R¹=R²=4-Cl, n=1) was flash-chromatographed on neutral silica gel using chloroform-methanol (98:2 by volume) as eluent. Mp 184°C (ethyl acetate).

### Example 52

### 5-[(2,3-Dihydroxypropoxy)methyl]-3-phenyl-3-(1H-imidazol-1-ylmethyl)-2-methylisoxazolidine (12: R¹=H, R² = CH₂CH(OH)CH₂OH)

A solution of 7.88 g (36.6 mmol) of 1-phenyl-2-(1H-imidazol-1-yl)-N-methylethanimine N-oxide (2: R¹ = H) [prepared by reacting 2-(1H-imidazol-1-yl)acetophenone (18.70 g, 0.10 mol), N-methylhydroxylamine hydrochloride (9.78 g, 0.117 mol), and K₂CO₃ (17.24 g, 0.125 mol) in 200 ml of ethanol] and 5.40 ml (5.77 g, 43.6 mmol) of 3-allyloxy-1,2-propandiol [11: R² = CH₂CH(OH)CH₂OH] in 150 ml of toluene is refluxed for 50 hours under a nitrogen atmosphere. Upon cooling to room temperature the solvent is removed under reduced pressure. The residual oil is crystallized from ethyl acetate to provide 6.21 g (49%) of compound 12 [R¹ = H, R² = CH₂CH(OH)CH₂OH], mp 99-104°C.

Anal. Calcd for C₁₈H₂₅N₃O₄: C, 62.23; H, 7.25; N, 12.10. Found: C, 62.15; H, 7.22; N, 12.02.

### Example 53

### 5-[(Cyclohexyloxy)methyl]-3-(4-chlorophenyl)-3-(1H-imidazol-1-ylmethyl)-2-methylisoxazolidine (12: R¹ = 4-Cl, R² = c-C₆H₁₁)

Derivative 12 (R¹ = 4-Cl, R² = c-C₆H₁₁) is prepared by a procedure similar to that described in Example 52 by reacting 1-(4-chlorophenyl)-2-(1H-imidazol-1-yl)-N-methylethanimine N-oxide (2: R¹ = 4-Cl) with allyl cyclohexyl ether (11: R² = c-C₆H₁₁). The resulting cis- and trans-diastereomeric mixture of compound 12 (R¹ = 4-Cl, R² = c-C₆H₁₁) is flash-chromatographed on neutral silica gel using a 99:1 by volume mixture of chloroform-methanol as eluent. Isomer A has a melting point of 122-126°C (ethyl acetate). Anal. Calcd for C₂₁H₂₈ClN₃O₂: C, 64.69; H, 7.24; Cl, 9.09; N, 10.78. Found: C, 64.78; H, 7.30; Cl, 8.91; N, 10.74.

### Example 54

### 5-{[[4-(1,1-Dimethylethyl)cyclohexyl]oxy]methyl}-3-(4-chlorophenyl)-3-(1H-imidazol-1-ylmethyl)-2-methylisoxazolidine (12: R¹ = 4-Cl, R² = c-C₆H₁₀C(CH₃)₃-4)

Derivative 12 [R¹ = 4-Cl, R² = c-C₆H₁₀C(CH₃)₃-4] is prepared by a procedure similar to that described in Example 52 by reacting 1-(4-chlorophenyl)-2-(1H-imidazol-1-yl)-N-methylethanimine N-oxide (2: R¹ = 4-Cl) with allyl 4-tert-butylcyclohexyl ether [11: R² = c-C₆H₁₀C(CH₃)₃-4]. The resulting cis- and trans-diastereomeric mixture of compound 12 [R¹ = 4-Cl, R² = c-C₆H₁₀C(CH₃)₃-4] is flash-chromatographed on neutral silica gel using chloroform-methanol (99:1 by volume) as eluent. Isomer A has a melting point of 145-148°C (ethyl acetate). Anal. Calcd for C₂₅H₂₆ClN₃O₂: C, 67.32; H, 8.14; Cl, 7.95; N, 9.42. Found: C, 67.44; H, 8.09; Cl, 8.32; N, 9.37.

### Example 55

### 5-{[(4-Chlorophenyl)methoxy]methyl}-3-(4-chlorophenyl)-3-(1H-imidazol-1-ylmethyl)-2-methylisoxazolidine (12: R¹ = 4-Cl, R² = CH₂C₆H₄Cl-4)

Derivative 12 (R¹ = 4-Cl, R² = CH₂C₆H₄Cl-4 is prepared by a procedure similar to that described in Example 52 by reacting 1-(4-chlorophenyl)-2-(1H-imidazol-1-yl)-N-methylethanimine N-oxide (2: R¹ = 4-Cl) with allyl 4-chlorobenzyl ether (11: R² = CH₂C₆H₄Cl-4). The resulting cis- and trans-diastereomeric mixture of the title compound (12: R¹ = 4-Cl, R² = CH₂C₆H₄Cl-4) is flash-chromatographed on neutral silica gel using chloroform-methanol (98:2 by volume) as eluent. Isomer A has a melting point of 97-100°C (ethyl acetate-hexane, 1:1 by volume). Anal. Calcd for C₂₂H₂₃Cl₂N₃O₂: C, 61.12; H, 5.36; Cl, 16.40; N, 9.72. Found: C, 61.04; H, 5.46; Cl, 16.56; N, 9.72.

### Example 56

### 5-(2-Hydroxyethyl)-3-(4-chlorophenyl)-3-(1H-imidazol-1-yl- methyl)-2-methylisoxazolidine (15: R¹ = 4-Cl)

Derivative 15 (R¹ = 4-Cl) is prepared by a procedure similar to that described in Example 52 by refluxing a solution of 16.98 g (68 mmol) of 1-(4-chlorophenyl)-2-(1H-imidazol-1-yl)-N-methylethanimine N-oxide (2: R¹ = 4-Cl) with 9.0 ml (7.49 g, 104 mmol) 3-buten-1-ol (14) in 150 ml of toluene for 28 hours under a nitrogen atmosphere. The reaction mixture is then cooled to room temperature. The resulting cis- and trans-diastereomeric mixture of compound 15 (R¹ = 4-Cl) is flash-chromatographed on neutral silica gel using chloroform-methanol (96:4 by volume) as eluent. Isomer A has a melting point of 125-130°C (ethyl acetate).

### Example 57

### 5-(Hydroxymethyl)-3-(4-chlorophenyl)-3-(1H-imidazol-1-ylmethyl)-2-methylisoxazolidine (12: R¹ = 4-Cl, R² = H)

Derivative 12 (R¹ = 4-Cl, R² = H) is prepared by a procedure similar to that described in Example 52 by refluxing a solution of 24.97 g (100 mmol) of 1-(4-chlorophenyl)-2-(1H-imidazol-1-yl)-N-methylethanimine N-oxide (2: R¹ = 4-Cl) with 14.0 ml (11.95 g, 200 mmol) of allyl alcohol (11: R² = H) in 300 ml of toluene for 19 hours under a nitrogen atmosphere. The reaction mixture is then cooled to room temperature and the solvent removed under reduced pressure. The resulting cis- and trans-diastereomeric mixture of compound 12 (R¹ = 4-Cl, R² = H) is flash-chromatographed on neutral silica gel using as eluent a 9:1 by volume mixture of chloroform-methanol. Isomer A has a melting point of 169-170°C (isopropanol). Anal. Calcd for C₁₅H₁₈ClN₃O₂: C, 58.54, H, 5.89; Cl, 11.52; N, 13.65. Found: C, 58.42; H, 5.88; Cl, 11.51; N, 13.57.

### Example 58

### 5-(Methoxymethyl)-3-(4-chlorophenyl)-3-(1H-imidazol-1-ylmethyl)-2-methylisoxazolidine (12: R¹ = 4-Cl, R² = CH₃)

5-(Hydroxymethyl)-3-(4-chlorophenyl)-3-(1H-imidazol-1-ylmethyl)-2-methylisoxazolidine (12: R¹ = 4-Cl, R² = H) (2.50 g, 8 mmol) is added to a suspension of 1.32 g (24 mmol) of 50% by weight sodium hydride in 40 ml anhydrous tetrahydrofuran, at 0°C under a nitrogen atmosphere. After stirring for 1 hour at 0°C, 1.28 g (9 mmol) of methyl iodide is added and the resulting suspension is heated to reflux and stirred for 20 hours. Upon cooling to room temperature, the reaction mixture is poured into 100 ml of water and then extracted with chloroform (3x75 ml). The combined organic extract is dried over anhydrous magnesium sulfate and the solvent removed under reduced pressure. The crude residue is flash-chromatographed on neutral silica gel using chloroform-methanol (98:2 by volume) as eluent. Following crystallization from ethyl acetate, 1.67 g (64%) of compound 12 (R¹ = 4-Cl, R² = CH₃) are obtained, mp 123-126°C. Anal. Calcd for C₁₆H₂₀ClN₃O₂: C, 59.72; H, 6.26; N, 13.06. Found: C, 59.71; H, 6.33; N, 12.98.

### Example 59

### 5-[(2-Propenoxy)methyl]-3-(4-chlorophenyl)-3-(1H-imidazol-1-ylmethyl)-2-methylisoxazolidine (12: R¹ = 4-Cl, R² = CH₂CH=CH₂)

Derivative 12 (R¹ = 4-Cl, R² = CH₂CH=CH₂) is prepared by a procedure similar to that described in Example 58 by reacting 5-(hydroxymethyl)-3-(4-chlorophenyl)-3-(1H-imidazol-1-ylmethyl)-2-methylisoxazolidine (12: R¹ = 4-Cl, R² = H) with allyl bromide. Compound 12 (R¹ = 4-Cl, R² = CH₂CH=CH₂) is isolated by flash-chromatography on neutral silica gel using as eluent a 98:2 by volume mixture of chloroform-methanol, mp 89-92°C (ethyl acetate). Anal. Calcd for C₁₈H₂₂ClN₃O₂: C, 62.15; H, 6.38; Cl, 10.19; N, 12.08. Found: C, 61.94; H, 6.35; Cl, 10.29; N, 11.95.

### Example 60

### 5-[(Methanesulfonyloxy)methyl]-3-(4-chlorophenyl)-3-(1H-imidazol-1-ylmethyl)-2-methylisoxazolidine (13: R¹ = 4-Cl)

Methanesulfonyl chloride (4.17 ml, 54 mmol) is added dropwise, at 0°C under a nitrogen atmosphere, to a solution of 12.50 g (41 mmol) of 5-(hydroxymethyl)-3-(4-chlorophenyl)-3-(1H-imidazol-1-ylmethyl)-2-methylisoxazolidine (12: R¹ = 4-Cl, R² = H) in 100 ml of anhydrous pyridine. After stirring for 4.5 hours at 0°C, the reaction mixture is warmed to room temperature and diluted with water. Then, the reaction mixture is neutralized by cautious addition of 12.04 g potassium carbonate and extracted with methylene chloride (3x75 ml). The combined organic extract is dried over anhydrous magnesium sulfate and the solvent is removed under reduced pressure leaving 14.98 g (95%) of compound 13 (R¹ = 4-Cl), mp 159-162°C (chloroform-petroleum ether, 1:1 by volume). Anal. Calcd for C₁₆H₂₀ClN₃O₄S: C, 49.80; H, 5.22; N, 10.89. Found: C, 49.46; H, 5.13; N, 10.81.

### Example 61

### 5-(Ethoxymethyl)-3-(4-chlorophenyl)-3-(1H-imidazol-1-ylmethyl)-2-methylisoxazolidine (12: R¹ = 4-Cl, R² = CH₂CH₃)

To a solution of sodium ethoxide in ethanol [prepared from 1.16 g (50 mmol) of sodium metal and 50 ml ethanol] is added under a nitrogen atmosphere 3.56 g (9.2 mmol) of 5-[(methanesulfonyloxy)methyl]-3-(4-chlorophenyl)-3-(1H-imidazol-1-ylmethyl)-2-methylisoxazolidine (13: R¹ = 4-Cl). The resulting solution is refluxed for 3 hours, then cooled to room temperature, poured into 200 ml ice-water, and extracted with chloroform (3x100 ml). The organic layer is dried over anhydrous magnesium sulfate and the solvent is removed under reduced pressure. The crude residue is flash-chromatographed on neutral silica gel using a 98:2 by volume mixture of chloroform-methanol as eluent, to give 1.82 g (59%) of compound 12 (R¹ = 4-Cl, R² = CH₂CH₃), mp 147-149°C (ethyl acetate). Anal. Calcd for C₁₇H₂₂ClN₃O₂: C, 60.80; H, 6.60; N, 12.51. Found: C, 60.86; H, 6.66; N, 12.48.

### Example 62

### 5-(n-Butoxymethyl)-3-(4-chlorophenyl)-3-(1H-imidazol-1-ylmethyl)-2-methylisoxazolidine (12: R¹ = 4-Cl, R² = (CH₂)₃CH₃)

Derivative 12 [R¹ = 4-Cl, R² = (CH₂)₃CH₃] is prepared by a procedure similar to that described in Example 61 by reacting 5-[(methanesulfonyloxy)methyl]-3-(4-chlorophenyl)-3-(1H-imidazol-1-ylmethyl)-2-methylisoxazolidine (13: R¹ = 4-Cl) with sodium n-butoxide. Compound 12 [R¹ = 4-Cl, R² = (CH₂)₃CH₃] is isolated by flash-chromatography on neutral silica gel using as eluent a 98:2 by volume mixture of chloroform-methanol, mp 53-56°C (ethyl acetate-petroleum ether, 1:1 by volume). Anal. Calcd for C₁₉H₂₆ClN₃O₂: C, 62.71; H, 7.20; Cl, 9.74; N, 11.55. Found: C, 62.56; H, 7.14; Cl, 9.82; N, 11.51.

### Example 63

### 5-(Acetyloxymethyl)-3-(4-chlorophenyl)-3-(1H-imidazol-1-ylmethyl)-2-methylisoxazolidine (18: R¹ = 4-Cl, R² = CH₃, n=1)

### Preparation of 5-(hydroxymethyl)-3-(4-chlorophenyl)-3-(1H-imidazol-1-ylmethyl)-2-methylisoxazolidine (17: R¹ = 4-Cl, n = 1):

A solution of 24.97 g (100 mmol) of 1-(4-chlorophenyl)-2-(1H-imidazol-1-yl)-N-methylethanimine N-oxide [prepared by reacting 2-(1H-imidazol-1-yl)-4ʹ-chloroacetophenone (22.05 g, 0.10 mol), N-methylhydroxylamine hydrochloride (10.65 g, 0.127 mol), and sodium bicarbonate (10.84 g, 0.129 mol) in 300 ml of ethanol] and 14.0 ml (11.95 g, 200 mmol) of allyl alcohol (16, n=1) in 300 ml of toluene is refluxed for 19 hours under a nitrogen atmosphere. The reaction mixture is then cooled to room temperature and the solvent removed under reduced pressure. The residual oil is crystallized from isopropanol to provide 4.80 g of compound 17 (R¹ = 4-Cl, n = 1).

Acetic anhydride (1.98 g, 0.0194 mol) is added to an ice-cold solution of 3.00 g (0.0097 mol) of 5-(hydroxymethyl)-3-(4-chlorophenyl)-3-(1H-imidazol-1-ylmethyl)-2-methylisoxazolidine (17: R¹ = 4-Cl, n=1) and 1.98 g (0.0194 mol) of triethylamine in 25 ml methylene chloride under a nitrogen atmosphere. The reaction is allowed to warm to room temperature, stirred for 18 hours, diluted to 100 ml with methylene chloride and extracted with saturated aqueous sodium bicarbonate (2x50 ml). The organic layer is dried over anhydrous magnesium sulfate and the solvent removed in vacuo, leaving a yellow oil which is crystallized from ethyl acetate, giving 2.01 g (59%) of compound 18 (R¹ = 4-Cl, R² = CH₃, n=1), melting at 119-121°C (ethyl acetate). Anal. Calcd. for C₁₇ H₂₀ClN₃O₃: C, 58.37; H, 5.76; N, 12.01; Cl, 10.13. Found: C, 58.26; H, 5.80; N, 11.90; Cl, 10.24.

### Example 64

### 5-[(Benzoyloxy)methyl]-3-(4-chlorophenyl)-3-(1H-imidazol-1-ylmethyl)-2-methylisoxazolidine (18: R¹ = 4-Cl, R² = C₆H₅, n = 1)

Compound 18 (R¹ = 4-Cl, R² = C₆H₅, n = 1) is prepared by a method similar to that described in Example 63 by reacting 5-(hydroxymethyl)-3-(4-chlorophenyl)-3-(1H-imidazol-1-ylmethyl)-2-methylisoxazolidine (17: R¹ = 4-Cl, n = 1) with benzoyl chloride. Flash-chromatography on neutral silica gel using a 98:2 by volume mixture of chloroform and methanol as eluent gives compound 18 (R¹ = 4-Cl, R² = C₆H₅, n = 1), melting at 95-102°C (ethyl acetate). Anal. Calcd. for C₂₂H₂₂ClN₃O₃: C, 64.15; H, 5.38; N, 10.20; Cl, 8.61. Found: C, 64.02; H, 5.48; N, 10.21; Cl, 8.88.

### Example 65

### 5-[[(4-Chlorobenzoyl)oxy]methyl]-3-(4-chlorophenyl)-3-(1H-imidazol-1-ylmethyl)-2-methylisoxazolidine (18: R¹ = 4-Cl, R² = C₆H₄Cl-4, n = 1)

Compound 18 (R¹ = 4-Cl, R² = C₆H₄Cl-4, n = 1) is prepared by a method similar to that described in Example 63 by reacting 5-(hydroxymethyl)-3-(4-chlorophenyl)-3-(1H-imidazol-1-ylmethyl)-2-methylisoxazolidine (17: R¹ = 4-Cl, n = 1) with 4-chlorobenzoyl chloride. Flash-chromatography on neutral silica gel using a 98:2 by volume mixture of chloroform and methanol as eluent gives compound 18 (R¹ = 4-Cl, R² = C₆H₄Cl-4, n = 1), melting at 122-125°C (ethyl acetate). Anal. Calcd. for C₂₂H₂₁Cl₂N₃O₃: C, 59.20; H, 4.74; N, 9.41; Cl, 15.89. Found: C, 59.06; H, 4.88; N, 9.32; Cl, 15.37.

### Example 66

### 5-[[(4-Nitrobenzoyl)oxy]methyl]-3-(4-chlorophenyl)-3-(1H-imidazol-1-ylmethyl)-2-methylisoxazolidine (18: R¹ = 4-Cl, R² = C₆H₄NO₂-4, n = 1)

Compound 18 (R¹ = 4-Cl, R² = C₆H₄NO₂-4, n = 1) is prepared by a method similar to that described in Example 63 by reacting 5-(hydroxymethyl)-3-(4-chlorophenyl)-3-(1H-imidazol-1-ylmethyl)-2-methylisoxazolidine (17: R¹ = 4-Cl, n = 1) with 4-nitrobenzoyl chloride. Flash-chromatography on neutral silica gel using a 98:2 by volume mixture of chloroform and methanol as eluent gives compound 18 (R¹ = 4-Cl, R² = C₆H₄NO₂-4, n = 1), melting at 187-190°C (ethyl acetate). Anal. Calcd. for C₂₂H₂₁ClN₄O₅: C, 57.84; H, 4.63; N, 12.26; Cl, 7.76. Found: C, 57.75; H, 4.65; N, 12.21; Cl, 7.64.

### Example 67

### 5-(2-Acetyloxyethyl)-3-(4-chlorophenyl)-3-(1H-imidazol-1-ylmethyl)-2-methylisoxazolidine (18: R¹ = 4-Cl, R² = CH₃, n = 2)

### Preparation of 5-(2-hydroxyethyl)-3-(4-chlorophenyl)-3-(1H-imidazol-1-ylmethyl)-2-methylisoxazolidine (17: R¹ = 4-Cl, n = 2):

A solution of 16.98g (68 mmol) of 1-(4-chlorophenyl)-2-(1H-imidazol-1-yl)-N-methylethanimine N-oxide and 9.0 ml (7.49 g, 104 mmol) of 3-buten-1-ol in 150 ml of toluene is refluxed for 28 hours under a nitrogen atmosphere. The reaction mixture is then cooled to room temperature and the solvent removed under reduced pressure. The residual oil is crystallized from ethyl acetate to provide 4.01 g of compound 17 (R¹ = 4-Cl, n = 2).

Compound 18 (R¹ = 4-Cl, R² = CH₃, n = 2) is prepared by a method similar to that described in Example 63 by reacting the 5-(2-hydroxyethyl)-3-(4-chlorophenyl)-3-(1H-imidazol-1-ylmethyl)-2-methylisoxazolidine (17: R¹ = 4-Cl, n = 2) with acetic anhydride. Flash-chromatography on neutral silica gel using a 97:3 by volume mixture of chloroform and methanol as eluent gives compound 18 (R¹ = 4-Cl, R² = CH₃, n = 2), melting at 97-98°C (ether). Anal. Calcd. for C₁₈H₂₂ClN₃O₃: C, 59.42; H, 6.09; N, 11.55; Cl, 9.74. Found: C, 59.36; H, 6.14; N, 11.55; Cl, 9.81.

Other esters 18 of the invention where R² includes substituted phenyl and where the substituents are lower alkyl and lower alkoxy are prepared according to the method of Example 64 by substituting for benzoyl chloride the aroyl chlorides,
4-methylbenzoyl chloride, bp 225°C
4-methoxybenzoyl chloride, mp 22°C,
3-methylbenzoyl chloride bp 86°C/5 mm,
and 3-methoxybenzoyl chloride, bp 123°C/5 mm.

Other esters 18 of the invention where n = 3 or 4 can be prepared according to the method of Example 63 by substituting for allyl alcohol the alkenols,
4-penten-1-ol, bp 134-137°C,
and 5-hexen-1-ol, bp 78°C/25 mm.

### Example 68

### 3-Phenyl-3-(1H-imidazol-1-ylmethyl)-2-methyl-5-{[N-phenyl-N-(phenylmethyl)amino]methyl}isoxazolidine (20: R¹ = R² = H, R³ = CH₂C₆H₅)

A solution of 5.3 g (24.6 mmol) of 1-phenyl-2-(1H-imidazol-1-yl)-N-methylethanimine N-oxide (2: R¹ = H) [prepared by reacting 2-(1H-imidazol-1-yl)acetophenone (5.88 g, 0.032 mol), N-methylhydroxylamine hydrochloride (3.17 g, 0.038 mol), and NaOAc (6.24 g, 0.076 mol) in 50 ml of ethanol] and 10.2 g (45.7 mmol) N-phenyl-N-(phenylmethyl)-2-propen-1-amine (19: R² = H, R³ = CH₂C₆H₅) [prepared by reacting 11.88 g (64.8 mmol) of N-benzylaniline with 8.4 ml (11.76 g, 97.2 mmol) of allyl bromide and 5.84 g (55.1 mmol) of sodium carbonate in 75 ml of 80% aqueous ethanol] in 150 ml of toluene is refluxed under a nitrogen atmosphere for 27 hours. Upon cooling to room temperature, the solvent is removed under reduced pressure. The residual dark oil is flash-chromatographed on neutral silica gel, using a mixture of chloroform-methanol (98:2 by volume) as eluent, to give 5.72 g (53%) of isomer A (20: R¹ = R² = H, R³ = CH₂C₆H₅). Mp 171-176°C (ethyl acetate). Anal. Calcd for C₂₈H₃₀N₄O: C, 76.68; H, 6.89; N, 12.77. Found: C, 76.03; H, 7.11; N, 12.76.

### Example 69

### 3-(4-Chlorophenyl)-3-(1H-imidazol-1-ylmethyl)-2-methyl-5-[(phenylamino)methyl]isoxazolidine (20: R¹ = 4-Cl, R² = R³ = H)

Derivative 20 (R¹ = 4-Cl, R² = R³ = H) is obtained by a procedure similar to that described in Example 68 by reacting 1-(4-chlorophenyl)-2-(1H-imidazol-1-yl)-N-methylethanimine N-oxide (2: R¹ = 4-Cl) with N-phenyl-2-propen-1-amine (19: R² = R³ = H). The product, which is a cis- and trans-diastereomeric mixture, is flash-chromatographed on neutral silica gel using chloroform-methanol (97:3 by volume) as eluent. Isomer A has a melting point of 159-160°C (ethyl acetate). Anal. Calcd for C₂₁H₂₃ClN₄O: C, 65.87; H, 6.05; N, 14.63; Cl, 9.26. Found: C, 65.77; H, 6.20; N, 14.42; Cl, 9.48. Isomer B has a melting point of 172-173°C (ethyl acetate).

### Example 70

### 3-(4-Chlorophenyl)-3-(1H-imidazol-1-ylmethyl)-2-methyl-5-{[4-chlorophenyl)amino]methyl}isoxazolidine (20: R¹ = R² = 4-Cl, R³ = H)

Derivative 20 (R¹ = R² = 4-Cl, R³ = H) is prepared by a procedure similar to that described in Example 68 by reacting 1-(4-chlorophenyl)-2-(1H-imidazol-1-yl)-N-methylethanimine N-oxide (2: R¹ = 4-Cl) with N-(4-chlorophenyl)-2-propen-1-amine (19: R² = 4-Cl, R³ = H). Compound 20 (R¹ = R² = 4-Cl, R³ = H) is flash-chromatographed on neutral silica gel using ethyl acetate as eluent. Isomer A has a melting point of 149-150.5°C (ethyl acetate). Anal. Calcd for C₂₁H₂₂Cl₂N₄O: C, 60.44; H, 5.31; N, 13.42; Cl, 16.99. Found: C, 60.40; H, 5.35; N, 13.27; Cl, 16.68.

### Example 71

### 3-(4-Chlorophenyl)-3-(1H-imidazol-1-ylmethyl)-2-methyl-5-{[(4-methoxyphenyl)amino]methyl}isoxazolidine (20: R¹ = 4-Cl, R² = 4-OCH₃, R³ = H)

Compound 20 (R¹ = 4-Cl, R² = 4-OCH₃, R³ = H) is prepared by a procedure similar to that described in Example 68 by reacting 1-(4-chlorophenyl)-2-(1H-imidazol-1-yl)-N-methylethanimine N-oxide (2: R¹ = 4-Cl) with N-(4-methoxyphenyl)-2-propen-1-amine (19: R²=4-OCH₃, R³=H). Derivative 20 (R¹ = 4-Cl, R² = 4-OCH₃, R³ = H) is flash-chromatographed on neutral silica gel using chloroform-methanol (99:1 by volume) as eluent. Isomer A has a melting point of 129-132°C (ethyl acetate). Anal. Calcd for C₂₂H₂₅ClN₄O₂: C, 63.99; H, 6.10; N, 13.57; Cl, 8.59. Found: C, 64.04; H, 6.10; N, 13.56; Cl, 8.58.

### Example 72

### 3-(4-Methoxyphenyl)-3-(1H-imidazol-1-ylmethyl)-2-methyl-5-{[(2-methylphenyl)amino]methyl}isoxazolidine (20: R¹ = 4-OCH₃, R² = 2-CH₃, R³ = H)

Derivative 20 (R¹ = 4-OCH₃, R² = 2-CH₃, R³ = H) is prepared by a procedure similar to that described in Example 68 by reacting N-(4-methoxyphenyl)-2-(1H-imidazol-1-yl)-N-methylethanimine N-oxide (2: 4-OCH₃) with N-(2-methylphenyl)-2-propen-1-amine (19: R² = 2-CH₃, R³ = H). Compound 20 (R¹ = 4-OCH₃), R² = 2-CH₃, R³ = H) is flash-chromatographed on neutral silica gel using chloroform-methanol (98:2 by volume) as eluent. Isomer A has a melting point of 110-111°C (ethyl acetate). Anal. Calcd for C₂₃H₂₈N₄O₂: C, 70.38; H, 7.19; N, 14.27. Found: C, 69.87; H, 7.11; N, 14.09.

### Example 73

### 3-(3,4-Dichlorophenyl)-3-(1H-imidazol-1-ylmethyl)-2-methyl-5-{[(4-nitrophenyl)amino]methyl}isoxazolidine (20: R¹ = 3,4-Cl₂,R² = 4-NO₂, R³ = H)

Compound 20 (R¹ = 3,4-Cl₂, R² = 4-NO₂, R³ = H) is prepared by a procedure similar to that described in Example 1 by reacting 1-(3,4-dichlorophenyl)-2-(1H-imidazol-1-yl)-N-methylethanimine N-oxide (2: R¹ = 3,4-Cl₂) with N-(4-nitrophenyl)-2-propen-1-amine (19: R² = 4-NO₂, R³ = H). Derivative 20 (R¹ = 3,4-Cl₂, R₂ = 4-NO₂, R³= H) is flash-chromatographed on neutral silica gel using chloroform-methanol (97:3 by volume) as eluent. Isomer A has a melting point of 133-134°C (ethyl acetate). Isomer B has a melting point of 147-148°C (ethyl acetate).

### Example 74

### 3-(3-Methylphenyl)-3-(1H-imidazol-1-ylmethyl)-2-methyl-5-[(N-phenyl-N-methylamino)methyl]isoxazolidine (20: R¹ = 3-CH₃, R² = H, R³ = CH₃)

Compound 20 (R¹ = 3-CH₃, R² = H, R³ = CH₃) is obtained by a procedure similar to that described in Example 68 by reacting 1-(3-methylphenyl)-2-(1H-imidazol-1-yl)-N-methylethanimine N-oxide (2: R¹ = 3-CH₃) with N-phenyl-N-methyl-2-propen-1-amine (19: R² = H, R³ = CH₃). Derivative 20 (R¹ = 3-CH₃, R² = H, R³ = CH₃) is flash-chromatographed on neutral silica gel using 98:2 by volume mixture of ethyl acetate-methanol as eluent. Isomer A has a melting point of 130-134°C (ethyl acetate).

### Example 75

### 5-(Phenylmethyl)-3-(4-chlorophenyl)-3-(1H-imidazol-1-ylmethyl)-2-methylisoxazolidine (22: R¹ = 4-Cl, R² = CH₂C₆H₅)

A solution of 7.04 g (0.0282 mol) of 1-(4-chlorophenyl)-2-(1H-imidazol-1-yl)-N-methylethanimine N-oxide (2: R¹ = 4-Cl) [prepared by reacting 2-(1H-imidazol-1-yl)-4ʹ-chloroacetophenone (45.05 g, 0.204 mol), N-methylhydroxylamine hydrochloride (20.93 g, 0.251 mol), and sodium acetate (41.13 g, 0.502 mol) and 550 ml of ethanol] and 3.95 g (0.0334 mol) of allylbenzene (21: R² = CH₂C₆H₅) in 100 ml toluene is refluxed for 30 hours under a nitrogen atmosphere. Upon cooling to room temperature, the solvent is removed in vacuo. The resulting cis- and trans-diastereomeric mixture of compound 22 (R¹ = 4-Cl, R² = CH₂C₆H₅) is flash-chromatographed on neutral silica gel using a 98:2 by volume mixture of chloroform and methanol as eluent to give 3.80 g (36%) of isomer A. Following crystallization from ether a melting point of 118-120°C is determined. Anal. Calcd. for C₂₁H₂₂ClN₃O: C, 68.56; H, 6.03, N. 11.42. Found: C, 68.66; H, 6.16; N, 11.51.

### Example 76

### 5-n-Hexyl-3-(4-chlorophenyl)-3-(1H-imidazol-1-ylmethyl)-2-methylisoxazolidine [22: R¹ = 4-Cl, R² = (CH₂)₅CH₃]

Compound 22 [R¹ = 4-Cl, R² = (CH₂)₅CH₃] is prepared by a method similar to that described in Example 75 by reacting 1-(4-chlorophenyl)-2-(1H-imidazol-1-yl)-N-methylethanimine N-oxide (2: R¹ = 4-Cl) with 1-octene [21: R² = (CH₂)₅CH₃]. The resulting cis- and trans-diastereomeric mixture of compound 22 [R¹ = 4-Cl, R² = (CH₂)₅CH₃] is flash-chromatographed on neutral silica gel using ethyl acetate as eluent. Isomer A has a melting point of 129-132°C (ethyl acetate) as its hydrochloride salt. Anal. Calcd. for C₂₀H₂₉Cl₂N₃O: C, 60.30; H, 7.34; N, 10.55; Cl, 17.80. Found: C, 60.27; H, 7.28; N, 10.50; Cl, 17.29.

### Example 77

### 5-n-Decyl-3-(4-chlorophenyl)-3-(1H-imidazol-1-ylmethyl)-2-methylisoxazolidine [22: R¹ = 4-Cl, R² = (CH₂)₉CH₃]

Compound 22 [R¹ = 4-Cl, R² = (CH₂)₉CH₃] is prepared by a method similar to that described in Example 75 by reacting 1-(4-chlorophenyl)-2-(1H-imidazol-1-yl)-N-methylethanimine N-oxide (2: R¹ = 4-Cl) with 1-dodecene [21: R² = (CH₂)₉CH₃]. The resulting cis- and trans-diastereomeric mixture of compound 22 [R¹ = 4-Cl, R² = (CH₂)₉CH₃] is flash-chromatographed on neutral silica gel using ethyl acetate as eluent. Isomer A has a melting point of 75-80°C (acetonitrile). Anal. Calcd. for C₂₄H₃₆ClN₃O: C, 68.96; H, 8.68; N, 10.05; Cl, 8.48. Found: C, 68.96; H, 8.65; N, 10.11; Cl, 8.64.

### Example 78

### 5-n-Tetradecyl-3-(4-chlorophenyl)-3-(1H-imidazol-1-ylmethyl)-2-methylisoxazolidine [22: R¹ = 4-Cl, R² = (CH₂)₁₃CH₃]

Compound 22 [R¹ = 4-Cl, R² = (CH₂)₁₃CH₃] is prepared by a method similar to that described in Example 75 by reacting 1-(4-chlorophenyl)-2-(1H-imidazol-1-yl)-N-methylethanimine N-oxide (2: R¹ = 4-Cl) with 1-hexadecene [21: R² = (CH₂)₁₃CH₃]. The resulting cis- and trans-diastereomeric mixture of compound 22 [R¹ = 4-Cl, R² = (CH₂)₁₃CH₃] is flash-chromatographed on neutral silica gel using ethyl acetate as eluent. Isomer A has a melting point of 82-86°C (ethyl acetate). Anal. Calcd. for C₂₈H₄₄ClN₃O: C, 70.93; H, 9.35; N, 8.86; Cl, 7.48. Found: C, 70.95; H, 9.43; N, 8.87; Cl, 7.76.

### Example 79

### 5-n-Hexadecyl-3-(4-chlorophenyl)-3-(1H-imidazol-1-ylmethyl)-2-methylisoxazolidine [22: R¹ = 4-Cl, R² (CH₂)₁₅CH₃]

Compound 22 [R¹ = 4-Cl, R² = (CH₂)₁₅CH₃] is prepared by a method similar to that described in Example 75 by reacting 1-(4-chlorophenyl)-2-(1H-imidazol-1-yl)-N-methylethanimine N-oxide (2, R¹ = 4-Cl) with 1-octadecene [21: R² = (CH₂)₁₅CH₃]. The resulting cis- and trans-diastereomeric mixture of compound 22 [R¹ = 4-Cl, R² = (CH₂)₁₅CH₃] is flash-chromatographed on neutral silica gel using ethyl acetate as eluent. Isomer A has a melting point of 86-90°C (ethyl acetate). Anal. Calcd. for C₃₀H₄₈ClN₃O: C, 71.75; H, 9.63; N, 8.37; Cl, 7.06. Found: C, 71.77; H, 9.62; N, 8.35; Cl, 7.15.

### Example 80

### 5-n-Octadecyl-3-(4-chlorophenyl)-3-(1H-imidazol-1-ylmethyl)-2-methylisoxazolidine [22: R¹ = 4-Cl, R² = (CH₂)₁₇CH₃]

Compound 22 [R¹ = 4-Cl, R² = (CH₂)₁₇CH₃] is prepared by a method similar to that described in Example 75 by reacting 1-(4-chlorophenyl)-2-(1H-imidazol-1-yl)-N-methylethanimine N-oxide (2, R¹ = 4-Cl) with 1-eicosene [21: R² = (CH₂)₁₇CH₃]. The resulting cis- and trans-diastereomeric mixture of compound 22 [R¹ = 4-Cl, R₂ = (CH₂)₁₇CH₃] is flash-chromatographed on neutral silica gel using a 98:2 by volume mixture of chloroform and methanol as eluent. Isomer A has a melting point of 81-86°C (ether). Anal. Calcd. for C₃₂H₅₂ClN₃O: C, 72.49; H, 9.89; N, 7.92; Cl, 6.69. Found: C, 72.57; H, 9.85; N, 7.87; Cl, 6.81.

### Example 81

### 5-(Cyclopentylmethyl)-3-(4-fluorophenyl)-3-(1H-imidazol-1-ylmethyl)-2-methylisoxazolidine [22: R¹ = 4-F, R² = CH₂C₅H₉(c)]

Compound 22 [R¹ = 4-F, R² = CH₂C₅H₉(c)] is prepared by a method similar to that described in Example 75 by reacting 1-(4-fluorophenyl)-2-(1H-imidazol-1-yl)-N-methylethanimine N-oxide (2: R¹ = 4-F) with allylcyclopentane [21: R² = CH₂C₅H₉(c)]. The resulting cis- and trans-diastereomeric mixture of compound 22 [R¹ = 4-F, R² = CH₂C₅H₉(c)] is flash-chromatographed on neutral silica gel using a 98:2 by volume mixture of chloroform and methanol as eluent. Isomer A has a melting point of 111-114°C (ethyl acetate). Anal. Calcd. for C₂₀H₂₆FN₃O: C, 69.94; H, 7.63; N, 12.23; F, 5.53. Found: C, 70.15; H, 7.54; N, 12.20; F, 5.19.

### Example 82

### 5-[2,2-Bis(ethoxycarbonyl)ethyl]-3-(4-fluorophenyl)-3-(1H- imidazol-1-ylmethyl)-2-methylisoxazolidine [22: R¹ = 4-F, R² = CH₂CH(CO₂Et)₂]

Compound 22 [R¹ = 4-F, R² = CH₂CH(CO₂Et)₂] is prepared by a method similar to that described in Example 75 by reacting 1-(4-fluorophenyl)-2-(1H-imidazol-1-yl)-N- methylethanimine N-oxide (2: R¹ = 4-F) with diethyl allylmalonate [21; R² = CH₂CH(CO₂Et)₂]. The resulting cis- and trans-diastereomeric mixture of compound 22 [R¹ = 4-F, R² = CH₂CH(CO₂Et)₂] is flash-chromatographed on neutral silica gel using a 99:1 by volume mixture of chloroform and methanol as eluent. Isomer A has a melting point of 75-78°C (ether). Isomer B has a melting point of 68-71°C (ether)., Anal. Calcd. for C₂₂H₂₈FN₃O₅: C, 60.96; H, 6.51; N, 9.69; F, 4.38. Found: C, 61.09; H, 6.58; N, 9.57; F, 4.29.

### Example 83

### 5-(2-trans-Phenylethenyl)-3-(4-chlorophenyl)-3-(1H-imidazol-1-ylmethyl)-2-methylisoxazolidine [22: R¹ = 4-Cl, R² = CH=CHC₆H₅(t)]

Compound 22 [R¹ = 4-Cl, R² = CH=CHC₆H₅(t)] is prepared by a method similar to that described in Example 75 by reacting 1-(4-chlorophenyl)-2-(1H-imidazol-1-yl)-N-methylethanimine N-oxide (2: R¹ = 4-Cl) with trans-1-phenyl-1,3-butadiene [21: R² = CH=CHC₆H₅(t)]. The resulting cis- and trans-diastereomeric mixture of compound 22 [R¹ = 4-Cl, R² = CH=CHC₆H₅(t)] is flash-chromatographed on neutral silica gel using a 98:2 by volume mixture of chloroform and methanol as eluent. Isomer A has a melting point of 101-104°C (ethyl acetate-hexane, 1:1 by volume). Anal. Calcd. for C₂₂H₂₂ClN₃O: C, 69.56; H, 5.84; N, 11.06; Cl, 9.33. Found: C, 69.36; H, 5.90; N, 11.02; Cl, 9.36.

### Example 84

### 5-(2-trans-Phenylethenyl)-3-(4-fluorophenyl)-3-(1H-imidazol-1-ylmethyl)-2-methylisoxazolidine [22: R¹ = 4-F, R² = CH=CHC₆H₅(t)]

Compound 22 [R¹ = 4-F, R² = CH=CHC₆H₅(t)] is prepared by a method similar to that described in Example 75 by reacting 1-(4-fluorophenyl)-2-(1H-imidazol-1-yl)-N-methylethanimine N-oxide (2: R¹ = 4-F) with trans-1-phenyl-1,3-butadiene [21: R² = CH=CHC₆H₅(t)]. The resulting cis- and trans-diastereomeric mixture of compound 22 [R¹ = 4-F, R² = CH=CHC₆H₅(t)] is flash-chromatographed on neutral silica gel using a 98:2 by volume mixture of chloroform and methanol as eluent. Isomer A has a melting point of 142-144°C (ethyl acetate-hexane, 1:1 by volume). Anal. Calcd. for C₂₂H₂₂FN₃O: C, 72.71; H, 6.10; N, 11.56; F, 5.23. Found: C, 72.90; H, 6.07; N, 11.56; F, 5.16.

Other compounds 22 of the invention where R² includes substituted phenylalkyl can be prepared according to the method of Example 75 by substituting for allylbenzene the following 3-(substituted phenyl)1-propenes,
3-(4-methylphenyl)-1-propene, bp. 183-186°C,
3-(4-methoxyphenyl)-1-propene, bp. 216°C,
and 3-(4-chlorophenyl)-1-propene, bp. 52°C/0.8 mm

The compounds 22 of the invention where R² includes substituted phenylalkenyl can be prepared according the method of Example 83 by substituting for trans-1-phenyl-1-3-butadiene the following 1-(substituted phenyl)-1,3-butadienes,
trans-1-(4-methylphenyl)-1,3-butadiene, mp 26°C,
trans-1-(4-methoxyphenyl)-1,3-butadiene, mp 46°C,
trans-1-(4-chlorophenyl)-1,3-butadiene, mp 18°C.

## Claims

1. A compound of the formula: 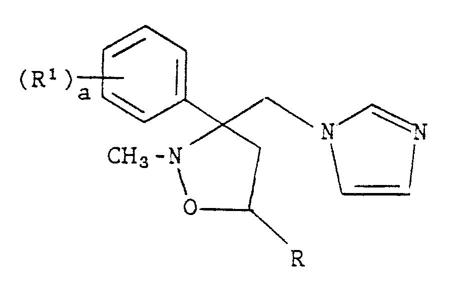 wherein;
a = 1 or 2,
R¹ is selected from hydrogen, lower alkyl, lower alkoxy, halogen, and combinations thereof, provided that the ortho position is hydrogen, and
R is selected from; 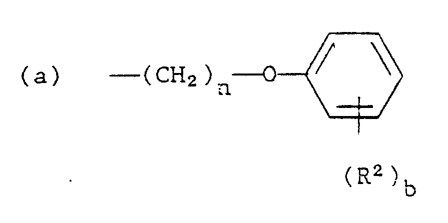 wherein;
b = 1 or 2,
n = 1 to 8, and
R² is selected from hydrogen, lower alkyl, lower alkoxy, halogen, trifluoromethyl, nitro, acetamido, and combinations thereof, 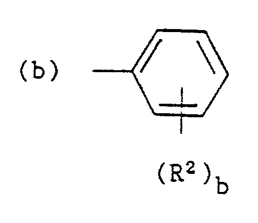 wherein;
b = 1 or 2, and
R² is selected from hydrogen, halogen, lower alkyl, lower alkoxy, and combinations thereof; 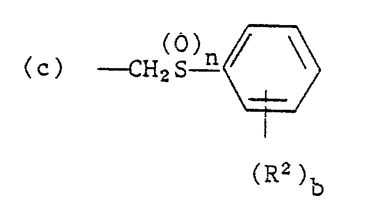 wherein;
n = 0 to 2,
b = 1 or 2, and
R² is selected from hydrogen, halogen, lower alkyl, lower alkoxy, nitro, and combinations thereof;
(d) ―(CH₂)ₙOR²
wherein;
n = 1 to 4, and
R² is selected from hydrogen, lower alkyl, mono- or dihydroxy-substituted lower alkyl, allyl, cyclohexyl, lower alkyl-substituted cyclohexyl, methanesulfonyl and (halophenyl)methyl, 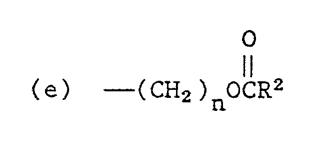
wherein;
n = 1 to 4, and
R² is selected from lower alkyl, phenyl, and mono- or disubstituted phenyl where the substituents on the phenyl ring are selected from halogen, lower alkyl, lower alkoxy, nitro, and combinations thereof, 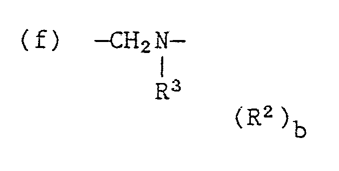 wherein;
b = 1 or 2,
R² is selected from hydrogen, lower alkyl, lower alkoxy, halogen, nitro, and combinations thereof, and R³ is selected from hydrogen, lower alkyl and benzyl, and
(g) phenylalkyl, substituted phenylalkyl, phenylalkenyl, substituted phenylalkenyl, C₁ to C₁₈ alkyl, (cycloalkyl)alkyl, and (alkoxycarbonyl)alkyl, where the phenyl substituents are selected from one or more of lower alkyl, lower alkoxy, and halogen including combinations thereof,
and pharmaceutically acceptable acid addition salts thereof, in the form of their enantiomers, or mixtures of their enantiomers including diastereoisomeric pairs of such enantiomers.

2. A compound of claim 1 wherein R is: 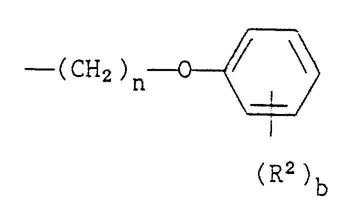 wherein;
b = 1 or 2,
n = 1 to 8, and
R² is selected from hydrogen, lower alkyl, lower alkoxy, halogen, trifluoromethyl, nitro, acetamido, and combinations thereof.

3. A compound of claim 1 wherein R is: 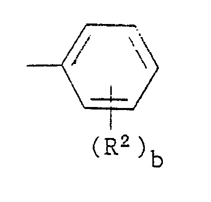 wherein;
b = 1 or 2, and
R² is selected from hydrogen, halogen, lower alkyl, lower alkoxy, and combinations thereof.

4. A compound of claim 1 wherein R is: 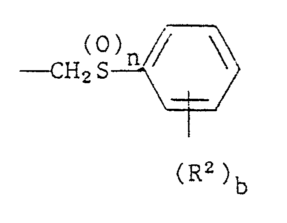 wherein;
n = 0 to 2,
b = 1 or 2, and
R² is selected from hydrogen, halogen, lower alkyl, lower alkoxy, nitro, and combinations thereof.

5. A compound of claim 1 wherein R is:
―(CH₂)ₙOR²
wherein;
n = 1 to 4, and
R² is selected from hydrogen, lower alkyl, mono- or dihydroxy-substituted lower alkyl, allyl, cyclohexyl, lower alkyl-substituted cyclohexyl, methanesulfonyl and (halophenyl)methyl.

6. A compound of claim 1 wherein R is: 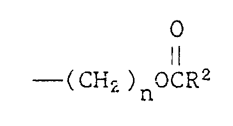 wherein; n = 1 to 4, and
R² is selected from lower alkyl, phenyl, and mono- or disubstituted phenyl where the substituents on the phenyl ring are selected from halogen, lower alkyl, lower alkoxy, nitro, and combinations thereof.

7. A compound of claim 1 wherein R is: 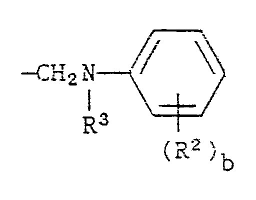 wherein;
b = 1 or 2,
R² is selected from hydrogen, lower alkyl, lower alkoxy, halogen, nitro, and combinations thereof, and
R³ is selected from hydrogen, lower alkyl and benzyl.

8. A compound of claim 1 wherein R is:
phenylalkyl, substituted phenylalkyl, phenylalkenyl, substituted phenylalkenyl, C₁ to C₁₈ alkyl, (cycloalkyl)alkyl, and (alkoxycarbonyl)alkyl wherein the phenyl substituents are selected from one or more of lower alkyl, lower alkoxy, and halogen including combinations thereof.

9. A compound according to claim 2 wherein the compound is 5-[(4-chlorophenoxy)methyl]-3-(4-chlorophenyl)-3-(1H-imidazol-1-ylmethyl)-2-methylisoxazolidine.

10. A compound according to claim 2 wherein the compound is 5-[(4-chlorophenoxy)methyl]-3-(4-fluorophenyl)-3-(1H-imidazol-1-ylmethyl)-2-methylisoxazolidine.

11. A compound according to claim 2 wherein the compound is 5-[(4-chlorophenoxy)methyl]-3-phenyl-3-(1H-imidazol-1-ylmethyl)-2-methylisoxazolidine.

12. A compound according to claim 2 wherein the compound is 5-{[3-(trifluoromethyl)phenoxy]methyl}-3-(4-chlorophenyl)-3-(1H-imidazol-1-ylmethyl)-2-methylisoxazolidine.

13. A compound according to claim 2 wherein the compound is 5-[(4-methoxyphenoxy)methyl]-3-(4-chlorophenyl)-3-(1H-imidazol-1-ylmethyl)-2-methylisoxazolidine.

14. A compound according to claim 2 wherein the compound is 5-[(2,4-dichlorophenoxy)methyl]-3-(4-chlorophenyl)-3-(1H-imidazol-1-ylmethyl)-2-methylisoxazolidine.

15. A compound according to claim 2 wherein the compound is 5-[(4-fluorophenoxy)methyl]-3-(4-chlorophenyl)-3-(1H-imidazol-1-ylmethyl)-2-methylisoxazolidine.

16. A compound according to claim 2 wherein the compound is 5-[(4-chloro-3-methylphenoxy)methyl]-3-(1H-imidazol-1-ylmethyl)-2-methyl-3-phenylisoxazolidine.

17. A compound according to claim 2 wherein the compound is 5-[(4-fluorophenoxy)methyl]-3-(4-fluorophenyl)-3-(1H-imidazol-1-ylmethyl)-2-methylisoxazolidine.

18. A compound according to claim 2 wherein the compound is the HCl or HNO₃ salt of 5-[(4-chlorophenoxy)methyl]-3-(4-chlorophenyl)-3-(1H-imidazol-1-ylmethyl)-2-methylisoxazolidine.

19. A compound according to claim 3 wherein the compound is 3-(4-chlorophenyl)-3-(1H-imidazol-1-ylmethyl)-2-methyl-5-phenylisoxazolidine.

20. A compound according to claim 3 wherein the compound is 3,5-bis-(4-chlorophenyl)-3-(1H-imidazol-1-ylmethyl)-2-methylisoxazolidine.

21. A compound according to claim 3 wherein the compound is 5-(2-chlorophenyl)-3-(4-chlorophenyl)-3-(1H-imidazol-1-ylmethyl)-2-methylisoxazolidine.

22. A compound according to claim 3 wherein the compound is 3-(4-chlorophenyl)-5-(2,6-dichlorophenyl)-3-(1H-imidazol-1-ylmethyl)-2-methylisoxazolidine.

23. A compound according to claim 3 wherein the compound is 3-(4-chlorophenyl)-3-(1H-imidazol-1-ylmethyl)-2-methyl-5-(4-methylphenyl)isoxazolidine.

24. A compound according to claim 4 wherein the compound is 3-(4-chlorophenyl)-3-(1H-imidazol-1-ylmethyl)-2-methyl-5-{[(4-methylphenyl)thio]methyl}isoxazolidine.

25. A compound according to claim 4 wherein the compound is 3-(4-chlorophenyl)-3-(1H-imidazol-1-ylmethyl)-2-methyl-5-{[(4-fluorophenyl)thio]methyl}isoxazolidine.

26. A compound according to claim 4 wherein the compound is 3-(4-chlorophenyl)-3-(1H-imidazol-1-ylmethyl)-2-methyl-5-{[(4-chlorophenyl)thio]methyl}isoxazolidine.

27. A compound according to claim 4 wherein the compound is 3-(4-chlorophenyl)-3-(1H-imidazol-2-ylmethyl)-2-methyl-5-{[(4-chlorophenyl)thio]methyl}isoxazolidine.

28. A compound according to claim 4 wherein the compound is 3-(4-fluorophenyl)-3-(1H-imidazol-1-ylmethyl)-2-methyl-5-{[(4-methylphenyl)thio]methyl}isoxazolidine.

29. A pharmaceutical composition characterized by the content of at least one of the compounds of claims 1 to 28 together with usual carriers.

## Claims (Claims for the following Contracting State(s): AT and ES:)

1. A process for preparing a compound having the formula: 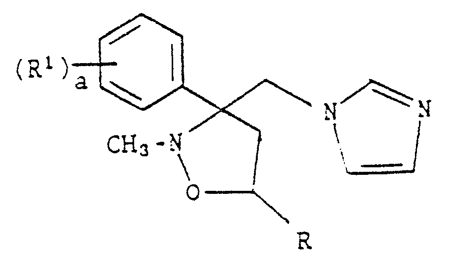 wherein:
a = 1 or 2,
R¹ is selected from hydrogen, lower alkyl, lower alkoxy, halogen, and combinations thereof, provided that the ortho position is hydrogen, and
R is selected from; 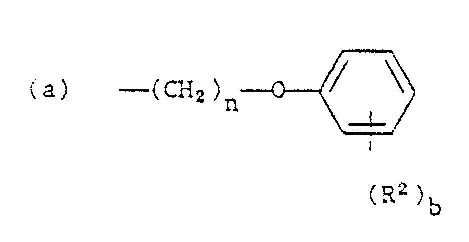 wherein:
b = 1 or 2,
n = 1 to 8, and
R² is selected from hydrogen, lower alkyl, lower alkoxy, halogen, trifluoromethyl, nitro, acetamido, and combinations thereof, 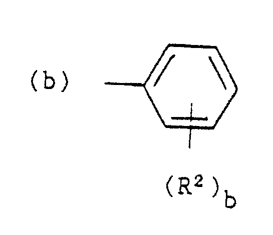 wherein:
b = 1 or 2, and
R² is selected from hydrogen, halogen, lower alkyl, lower alkoxy, and combinations thereof; 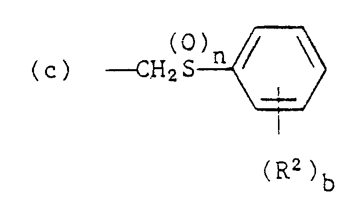 wherein:
n = 0 to 2,
b = 1 or 2, and
R² is selected from hydrogen, halogen, lower alkyl, lower alkoxy, nitro, and combinations thereof;
(d) ―(CH₂)ₙOR²
wherein:
n = 1 to 4, and
R² is selected from hydrogen, lower alkyl, mono- or dihydroxy-substituted lower alkyl, allyl, cyclohexyl, lower alkyl-substituted cyclohexyl, methanesulfonyl and (halophenyl)methyl, 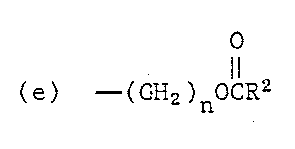
wherein:
n = 1 to 4, and
R² is selected from lower alkyl, phenyl, and mono- or disubstituted phenyl where the substituents on the phenyl ring are selected from halogen, lower alkyl, lower alkoxy, nitro, and combinations thereof, 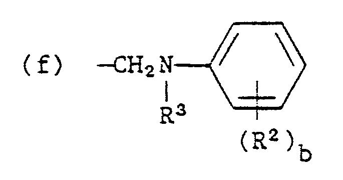 wherein:
b = 1 or 2,
R² is selected from hydrogen, lower alkyl, lower alkoxy, halogen, nitro, and combinations thereof, and R³ is selected from hydrogen, lower alkyl and benzyl, and
(g) phenylalkyl, substituted phenylalkyl, phenylalkenyl, substituted phenylalkenyl, C₁ to C₁₈ alkyl, (cycloalkyl)alkyl, and (alkoxycarbonyl)alkyl, wherein the phenyl substituents are selected from one or more of lower alkyl, lower alkoxy, and halogen including combinations thereof,
and pharmaceutically acceptable acid addition salts thereof, in the form of their enantiomers, or mixtures of their enantiomers including diastereoisomeric pairs of such enantiomers, comprising reacting a nitrone of the formula: 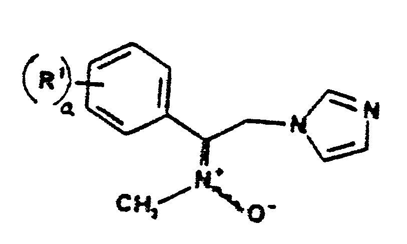 in which (R¹)ₐ is as defined above,
(1) when R is (a) above, with either an alkyl phenyl ether of the formula: 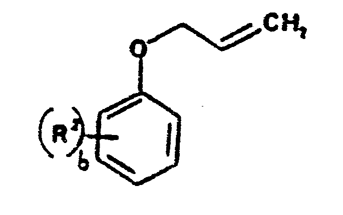 or a 1-(w-alkenyloxy)benzene of the formula: 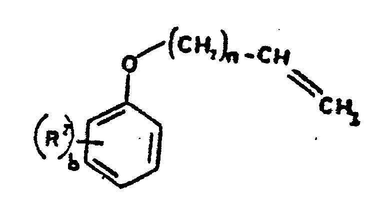 in which (R²)_{b} is as defined in (a) above,
(2) when R is (b) above, with a styrene of the formula: 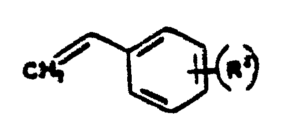 in which (R²)_{b} is as defined in (b) above,
(3) when R is (c) above, with an allyl (substituted phenyl) sulfide of the formula: 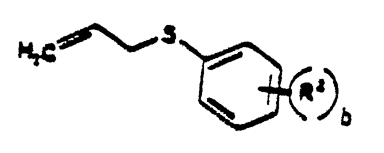 in which (R²)_{b} is as defined in (c) above and the sulfur could be oxidized,
(4) when R is (d) above, with 1-alkene alcohol or ether derivatives of the formulae, 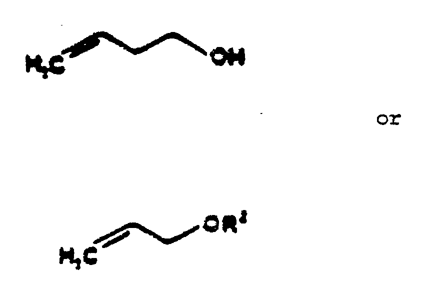 and further treating the ether compounds of (d) with methane sulfonyl chloride to prepare the methanesulfonyloxy derivatives,
(5) when R is (e) above, 1-alkene alcohols of the formula: 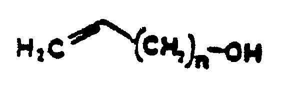 Where n is as defined in (e) above to prepare the alkanol compounds and further treating the alkanol compounds with acetic anhydride or an allyl chloride to provide the esters,
(6) when R is (f) above, 3-(phenylamino)prop-1-enes of the formula: 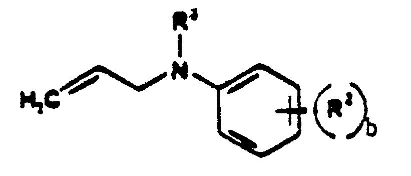 where (R²)_{b} is as defined in (f) above, and
(7) when R is (g) above with a 1-alkene derivative, having 3 to 21 carbons in the carbon chain, of the formula: 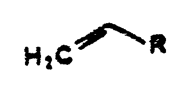 and optionally converting the resulting compound into its pharmaceutically acceptable acid addition salt.

2. The process of claim 1 for the production of a compound wherein R is: 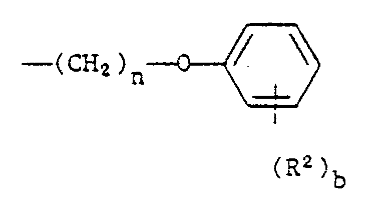 wherein:
b = 1 or 2,
n = 1 to 8, and
R² is selected from hydrogen, lower alkyl, lower alkoxy, halogen, trifluoromethyl, nitro, acetamido, and combinations thereof.

3. The process of claim 1 for the production of a compound wherein R is: 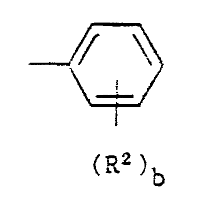 wherein:
b = 1 or 2, and
R² is selected from hydrogen, halogen, lower alkyl, lower alkoxy, and combinations thereof.

4. The process of claim 1 for the production of a compound wherein R is: 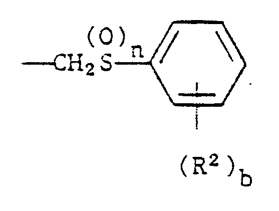 wherein;
n = 0 to 2,
b = 1 or 2, and
R² is selected from hydrogen, halogen, lower alkyl, lower alkoxy, nitro, and combinations thereof.

5. The process of claim 1 for the production of a compound wherein R is:
―(CH₂)ₙOR²
wherein:
n = 1 to 4, and
R² is selected from hydrogen, lower alkyl, mono- or dihydroxy-substituted lower alkyl, allyl, cyclohexyl, lower alkyl-substituted cyclohexyl, methanesulfonyl and (halophenyl)methyl.

6. The process of claim 1 for the production of a compound wherein R is: 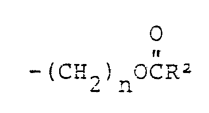 wherein: n = 1 to 4, and
R² is selected from lower alkyl, phenyl, and mono- or disubstituted phenyl where the substituents on the phenyl ring are selected from halogen, lower alkyl, lower alkoxy, nitro, and combinations thereof.

7. The process of claim 1 for the production of a compound wherein R is: 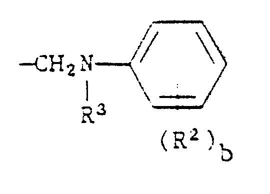 wherein:
b = 1 or 2,
R² is selected from hydrogen, lower alkyl, lower alkoxy, halogen, nitro, and combinations thereof, and
R³ is selected from hydrogen, lower alkyl and benzyl.

8. The process of claim 1 for the production of a compound wherein R is:
phenylalkyl, substituted phenylalkyl, phenylalkenyl, substituted phenylalkenyl, C₁ to C₁₈ alkyl, (cycloalkyl)alkyl, and (alkoxycarbonyl)alkyl wherein the phenyl substituents are selected from one or more of lower alkyl, lower alkoxy, and halogen including combinations thereof.

9. The process of claim 1 for the production of the compound 5-[(4-chlorophenoxy)methyl]-3-(4-chlorophenyl)-3-(1H-imidazol-1-ylmethyl)-2-methylisoxazolidine.

10. The process of claim 1 for the production of the compound 5-[(4-chlorophenoxy)methyl]-3-(4-fluorophenyl)-3-(1H-imidazol-1-ylmethyl)-2-methylisoxazolidine.

11. The process of claim 1 for the production of the compound 5-[(4-chlorophenoxy)methyl]-3-phenyl-3-(1H-imidazol-1-ylmethyl)-2-methylisoxazolidine.

12. The process of claim 1 for the production of the compound 5-{[3-(trifluoromethyl)phenoxy]methyl}-3-(4-chlorophenyl)-3-(1H-imidazol-1-ylmethyl)-2-methylisoxazolidine.

13. The process of claim 1 for the production of the compound 5-[(4-methoxyphenoxy)methyl]-3-(4-chlorophenyl)-3- (1H-imidazol-1-ylmethyl)-2-methylisoxazolidine.

14. The process of claim 1 for the production of the compound 5-[(2,4-dichlorophenoxy)methyl]-3-(4-chlorophenyl)-3-(1H-imidazol-1-ylmethyl)-2-methylisoxazolidine.

15. The process of claim 1 for the production of the compound 5-[(4-fluorophenoxy)methyl]-3-(4-chlorophenyl)-3-(1H-imidazol-1-ylmethyl)-2-methylisoxazolidine.

16.The process of claim 1 for the production of the compound 5-[(4-chloro-3-methylphenoxy)methyl]-3-(1H-imidazol-1-ylmethyl)-2-methyl-3-phenylisoxazolidine.

17. The process of claim 1 for the production of the compound 5-[(4-fluorophenoxy)methyl]-3-(4-fluorophenyl)-3-(1H-imidazol-1-ylmethyl)-2-methylisoxazolidine.

18. The process of claim 1 for the production of the HCl or HNO₃ salt of 5-[(4-chlorophenoxy)methyl]-3-(4-chlorophenyl)-3-(1H-imidazol-1-ylmethyl)-2-methylisoxazolidine.

19. The process of claim 1 for the production of the compound 3-(4-chlorophenyl)-3-(1H-imidazol-1-ylmethyl)-2-methyl-5-phenylisoxazolidine.

20. The process of claim 1 for the production of the compound 3,5-bis-(4-chlorophenyl)-3-(1H-imidazol-1-ylmethyl)-2-methylisoxazolidine.

21. The process of claim 1 for the production of the compound 5-(2-chlorophenyl)-3-(4-chlorophenyl)-3-(1H-imidazol-1-ylmethyl)-2-methylisoxazolidine.

22. The process of claim 1 for the production of the compound 3-(4-chlorophenyl)-5-(2,6-dichlorophenyl)-3-(1H- imidazol-1-ylmethyl)-2-methylisoxazolidine.

23. The process of claim 1 for the production of the compound 3-(4-chlorophenyl)-3-(1H-imidazol-1-ylmethyl)-2-methyl-5-(4-methylphenyl)isoxazolidine.

24. The process of claim 1 for the production of the compound 3-(4-chlorophenyl)-3-(1H-imidazol-1-ylmethyl)-2-methyl-5-{[(4-methylphenyl)thio]methyl}isoxazolidine.

25. The process of claim 1 for the production of the compound 3-(4-chlorophenyl)-3-(1H-imidazol-1-ylmethyl)-2-methyl-5-{[(4-fluorophenyl)thio]methyl}isoxazolidine.

26. The process of claim 1 for the production of the compound 3-(4-chlorophenyl)-3-(1H-imidazol-1-ylmethyl)-2-methyl-5-{[(4-chlorophenyl)thio]methyl}isoxazolidine.

27. The process of claim 1 for the production of the compound 3-(4-chlorophenyl)-3-(1H-imidazol-2-ylmethyl)-2-methyl-5-{[(4-chlorophenyl)thio]methyl}isoxazolidine.

28. The process of claim 1 for the production of the compound 3-(4-fluorophenyl)-3-(1H-imidazol-1-ylmethyl)-2-methyl-5-{[(4-methylphenyl)thio]methyl}isoxazolidine.
